# EUROPEAN PATENT APPLICATION

(11) **EP 3 825 333 A1**
(43) Date of publication of application: **26.05.2021**
(21) Application number: 19831301.7
(22) Date of filing: 05.07.2019
(51) Int. Cl.: C07K 16/32, C07K 16/00, A61K 39/395, A61P 35/00

(54) **LOW FUNCTIONAL ADCC/CDC MONOCLONAL ANTIBODY, PREPARATION METHOD THEREFOR AND USE THEREOF**

(30) Priority: 06.07.2018 CN 201810738565; 14.08.2018 CN 201810924249; 07.05.2019 CN 201910376852
(71) Applicant: Abmax Biopharmaceuticals, Beijing 101111 (CN)
(72) Inventor: SUN, Le, Beijing 101111 (CN); LI, Maohua, Beijing 101111 (CN); REN, Wenlin, Beijing 101111 (CN)
(74) Representative: f & e patent
(86) International application number: PCT/CN2019/094928
(87) International publication number: WO 2020/007368

(57) **Abstract**

The present invention provides De-ADCC/CDC activity monoclonal antibodies and their generation method and applications. The present invention modified the antibody sequences of multi-target sites such as PD-L1, PD-1, CTLA4, TNF- α, PCSK9, NGF, CD47 and C5. A flexible amino acid sequence was inserted between the heavy chain CDR3 and CH2 regions of IgG1 antibody to block the physical stress signal transmit from antigen-bound Fab to its Fc region to reduce the ADCC/CDC activity while not significantly increasing the formation of the polymers. The modified antibodies in the present invention and the original antibodies target the same sites, but the antibody subtype or glycosylation state is different. For the modified antibodies in the present invention, the stability is significantly improved or/and immunogenicity is significantly reduced, and the half-life is prolonged. The modified antibodies eliminate the activity of ADCC/CDC. Therefore, the antibody modified by the technology of the present invention can effectively avoid side effects caused by the activation of ADCC/CDC and other Fc-mediated functions and improve the curative effect of antibody drugs. It has excellent clinical application value and is suitable for promotion.

Sequence Listing

<110>  AbMax Biopharmaceutical Co., LTD
<120>  De-ADCC/CDC functions of antibodies and their applications
<130>  KHP199810071.6
<160>  10
<170>  SIPOSequenceListing 1.0

<210>  1
<211>  448
<212>  PRT
<213>  Artificial Sequence

<400>  1
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Arg Arg His Trp Pro Gly Gly Phe Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
        275                 280                 285
Lys Thr Lys Pro Arg Glu Glu Gln Tyr Ala Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Glu Glu Met Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp

385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445

<210>  2
<211>  214
<212>  PRT
<213>  Artificial Sequence

<400>  2
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Ser Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Leu Tyr Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Leu Tyr His Pro Ala
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  3
<211>  452
<212>  PRT
<213>  Artificial Sequence

<400>  3
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Arg Arg His Trp Pro Gly Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Leu Gly Gly Gly Gly
225                 230                 235                 240
Ser Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Glu Glu Met Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450

<210>  4
<211>  454
<212>  PRT
<213>  Artificial Sequence

<400>  4
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Thr Tyr Tyr Ala Asp Ser Val
    50                  55                  60
Lys Gly Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Thr
    210                 215                 220
His Thr Cys Pro Pro Cys Pro Ala Pro Glu Leu Gly Gly Ser Gly Gly
225                 230                 235                 240
Ser Leu Gly Gly Pro Ser Val Phe Leu Phe Pro Pro Lys Pro Lys Asp
                245                 250                 255
Thr Leu Met Ile Ser Arg Thr Pro Glu Val Thr Cys Val Val Val Asp
            260                 265                 270
Val Ser His Glu Asp Pro Glu Val Lys Phe Asn Trp Tyr Val Asp Gly
        275                 280                 285
Val Glu Val His Asn Ala Lys Thr Lys Pro Arg Glu Glu Gln Tyr Asn
    290                 295                 300
Ser Thr Tyr Arg Val Val Ser Val Leu Thr Val Leu His Gln Asp Trp
305                 310                 315                 320
Leu Asn Gly Lys Glu Tyr Lys Cys Lys Val Ser Asn Lys Ala Leu Pro
                325                 330                 335
Ala Pro Ile Glu Lys Thr Ile Ser Lys Ala Lys Gly Gln Pro Arg Glu
            340                 345                 350
Pro Gln Val Tyr Thr Leu Pro Pro Ser Arg Glu Glu Met Thr Lys Asn
        355                 360                 365
Gln Val Ser Leu Thr Cys Leu Val Lys Gly Phe Tyr Pro Ser Asp Ile
    370                 375                 380
Ala Val Glu Trp Glu Ser Asn Gly Gln Pro Glu Asn Asn Tyr Lys Thr
385                 390                 395                 400
Thr Pro Pro Val Leu Asp Ser Asp Gly Ser Phe Phe Leu Tyr Ser Lys
                405                 410                 415
Leu Thr Val Asp Lys Ser Arg Trp Gln Gln Gly Asn Val Phe Ser Cys
            420                 425                 430
Ser Val Met His Glu Ala Leu His Asn His Tyr Thr Gln Lys Ser Leu
        435                 440                 445
Ser Leu Ser Pro Gly Lys
    450

<210>  5
<211>  447
<212>  PRT
<213>  Artificial Sequence

<400>  5
Gln Val Gln Leu Val Gln Ser Gly Val Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Tyr Met Tyr Trp Val Arg Gln Ala Pro Gly Gln Gly Leu Glu Trp Met
        35                  40                  45
Gly Gly Ile Asn Pro Ser Asn Gly Gly Thr Asn Phe Asn Glu Lys Phe
    50                  55                  60
Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95
Ala Arg Arg Asp Tyr Arg Phe Asp Met Gly Phe Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445

<210>  6
<211>  218
<212>  PRT
<213>  Artificial Sequence

<400>  6
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Gly Val Ser Thr Ser
            20                  25                  30
Gly Tyr Ser Tyr Leu His Trp Tyr Gln Gln Lys Pro Gly Gln Ala Pro
        35                  40                  45
Arg Leu Leu Ile Tyr Leu Ala Ser Tyr Leu Glu Ser Gly Val Pro Ala
    50                  55                  60
Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95
Asp Leu Pro Leu Thr Phe Gly Gly Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  7
<211>  454
<212>  PRT
<213>  Artificial Sequence

<400>  7
Gln Val Gln Leu Val Gln Ser Gly Val Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Tyr Met Tyr Trp Val Arg Gln Ala Pro Gly Gln Gly Leu Glu Trp Met
        35                  40                  45
Gly Gly Ile Asn Pro Ser Asn Gly Gly Thr Asn Phe Asn Glu Lys Phe
    50                  55                  60
Lys Asn Arg Val Thr Leu Thr Thr Asp Ser Ser Thr Thr Thr Ala Tyr
65                  70                  75                  80
Met Glu Leu Lys Ser Leu Gln Phe Asp Asp Thr Ala Val Tyr Tyr Cys
                85                  90                  95
Ala Arg Arg Asp Tyr Arg Phe Asp Met Gly Phe Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Gly Gly Ser Gly Pro Ser Val Phe Leu Phe Pro Pro Lys Pro Lys Asp
                245                 250                 255
Thr Leu Met Ile Ser Arg Thr Pro Glu Val Thr Cys Val Val Val Asp
            260                 265                 270
Val Ser His Glu Asp Pro Glu Val Lys Phe Asn Trp Tyr Val Asp Gly
        275                 280                 285
Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320
Leu Asn Gly Lys Glu Tyr Lys Cys Lys Val Ser Asn Lys Ala Leu Pro
                325                 330                 335
Ala Pro Ile Glu Lys Thr Ile Ser Lys Ala Lys Gly Gln Pro Arg Glu
            340                 345                 350
Pro Gln Val Tyr Thr Leu Pro Pro Ser Arg Glu Glu Met Thr Lys Asn
        355                 360                 365
Gln Val Ser Leu Thr Cys Leu Val Lys Gly Phe Tyr Pro Ser Asp Ile
    370                 375                 380
Ala Val Glu Trp Glu Ser Asn Gly Gln Pro Glu Asn Asn Tyr Lys Thr
385                 390                 395                 400
Thr Pro Pro Val Leu Asp Ser Asp Gly Ser Phe Phe Leu Tyr Ser Lys
                405                 410                 415
Leu Thr Val Asp Lys Ser Arg Trp Gln Gln Gly Asn Val Phe Ser Cys
            420                 425                 430
Ser Val Met His Glu Ala Leu His Asn His Tyr Thr Gln Lys Ser Leu
        435                 440                 445
Ser Leu Ser Pro Gly Lys
    450

<210>  8
<211>  448
<212>  PRT
<213>  Artificial Sequence

<400>  8
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Thr Met His Trp Val Arg Gln Ala Pro Gly Lys Gly Leu Glu Trp Val
        35                  40                  45
Thr Phe Ile Ser Tyr Asp Gly Asn Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Ile Tyr Tyr Cys
                85                  90                  95
Ala Arg Thr Gly Trp Leu Gly Pro Phe Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Arg Val Glu Pro Lys Ser Cys Asp Lys Thr
    210                 215                 220
His Thr Cys Pro Pro Cys Pro Ala Pro Glu Leu Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Asp Glu Leu Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445

<210>  9
<211>  215
<212>  PRT
<213>  Artificial Sequence

<400>  9
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Gly Ser Ser
            20                  25                  30
Tyr Leu Ala Trp Tyr Gln Gln Lys Pro Gly Gln Ala Pro Arg Leu Leu
        35                  40                  45
Ile Tyr Gly Ala Phe Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95
Trp Thr Phe Gly Gln Gly Thr Lys Val Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210>  10
<211>  452
<212>  PRT
<213>  Artificial Sequence

<400>  10
Gln Val Gln Leu Val Glu Ser Gly Gly Gly Val Val Gln Pro Gly Arg

1               5                   10                  15
Ser Leu Arg Leu Ser Cys Ala Ala Ser Gly Phe Thr Phe Ser Ser Tyr
            20                  25                  30
Thr Met His Trp Val Arg Gln Ala Pro Gly Lys Gly Leu Glu Trp Val
        35                  40                  45
Thr Phe Ile Ser Tyr Asp Gly Asn Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Ile Tyr Tyr Cys
                85                  90                  95
Ala Arg Thr Gly Trp Leu Gly Pro Phe Asp Tyr Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Arg Val Glu Pro Lys Ser Cys Asp Lys Thr
    210                 215                 220
His Thr Cys Pro Pro Cys Pro Ala Pro Glu Leu Leu Gly Gly Gly Gly
225                 230                 235                 240
Ser Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450

## Description

### CROSS-REFERENCE TO RELATED APPLICATION

This application claims the benefit of the Chinese Application No. 201810738565.8, filed on July 6, 2018 of the patent name for "De-ADCC/CDC function anti-PD-L1 human monoclonal antibody and its application", the Chinese Application No. 20181092424249.x, filed on 14 August 2018 of the patent name for "IgG1 subtype De-ADCC/CDC function antibody against human programmed death receptor PD-1", and the Chinese Application No. 201910376852.3, filed on May 7, 2019 of the patent name for "De-ADCC/CDC functional antibody of IgG1 subtype against human CTLA-4", and the entire disclosure of which are incorporated herein by reference.

### FIELD OF THE INVENTION

The present invention relates to generation and application of therapeutic antibody, and mainly involves a method by insertion of a flexible sequence in IgG heavy chain to reduce the ADCC/CDC functions of antibodies.

### BACKGROUND OF THE INVENTION

Antibody-dependent cell-mediated cytotoxicity (ADCC) refers to the cell killing effect on the target cell by the IgG Fc receptor-expressing NK cells, macrophages and neutrophils once the effector cells binds to the Fc of IgG antibody which bound the target tumor cells or virus-infected cells. NK cells are the main cells that play the key role of ADCC. In the process of the ADCC, the antibody first specifically binds to the corresponding antigen on the target cells, then the conformation of the antibody will change and expose the binding site(s) for Fc receptors, which in turn will recruit the Fc receptor expressing effector cells such as NK cells, and activate the NK cells to release granzyme and other cytokines to kill the cells locally. The binding of the antibody on the target cells is the specific, but the killing effect of the NK cells to the target cells is nonspecific.

Complement dependent cytotoxicity (CDC) refers to the activation of the complement system, the formation of MAC on the surface of target cells, resulting in target cell lysis.

Complement can lead to the lysis of many kinds of bacteria and other pathogenic organisms and is an important defense mechanism for the body to resist pathogen infection. Especially, it plays an important role in preventing the infection of gram-negative bacteria. In some cases, the complement system can cause tissue or cell damage and is involved in the pathogenesis of hypersensitivity and autoimmune diseases.

Antibody-dependent cell phagocytosis (ADCP) refers to the cell phagocytosis of target cell by the IgG Fc receptor-expressing macrophages once the effector cells binds to the Fc of IgG antibody which bound the target tumor cells or virus-infected cells.

The application of antibody drugs is not limited to infectious diseases and tumors. In order to avoid the adverse ADCC and CDC functions induced by antibody binding to target proteins/cells/tissues, the main methods currently adopted include 1) the mutation of amino acid N (297) to A in the antibody heavy chain to remove the glycosylation or 2) switch the antibody subtype from IgG1 to IgG2 or IgG4. However, the aglycosylation of the antibody heavy chain resulted in excessive polymers in the product, significantly enhanced immunogenicity of the antibody drug, and lead a high incidence of the development of anti-drug antibody (ADA) in patients after repeated administration. Antibodies of IgG2 subtype can still induce monocyte-mediated ADCC and macrophage-mediated ADCP by binding to FcRIIa. In addition, there are additional Cys residues in the upstream of hinge region of IgG2, so disulfide isomers may be formed to affect the conformation and function of IgG2 molecules. By changing the heavy chain of the antibody to IgG4 subtype, the heavy chain of IgG4 subtype will automatically exchange with that of other IgG4 antibodies in the blood of the patients, forming semi-molecular and bi-specific antibodies. IgG4 still retains the ADCP activity. Meanwhile, the half-life of IgG2 or IgG4 isoform antibody is significantly shorter than IgG1 isoform.

PD-1 is mainly expressed in activated T cells and B cells, and its function is to suppress the activation of immune cells to avoid excessive T/B cell activation, which can cause autoimmune diseases. This is a normal self-stabilizing mechanism of the immune system. However, tumor microenvironment will induce the infiltrated T cells to overexpress PD-1 molecules, and tumor cells will overexpress PD-1 ligands, such as PD-L1 and PD-L2, leading to the continuous activation of PD-1 pathway in tumor microenvironment, and the T cells will be inhibited and unable to kill tumor cells.

Both PD-1 and PD-L1 antibodies can block this pathway, partially restoring the function of T cells so that they can continue to kill tumor cells. On May 18, 2016, the FDA approved Atezolizumab(trade name: Tecentriq) of Genentech inc. injection for the treatment of adult patients with locally advanced or metastatic urothelial carcinoma who have disease progression during or following platinum-containing chemotherapy or have disease progression within 12 months of neoadjuvant or adjuvant treatment with platinum-containing chemotherapy. But in order to avoid the adverse ADCC and other functions, Genentech's Atezolizumab used the aglycosylation mutation, which resulted in excessive amounts of polymer in the antibody drug, lead to remarkable increase of drug immunogenicity, and the anti-drug antibodies (ADA) rate of patients after multiple administration is as high as 41.5%.

On September 4, 2014, the FDA granted accelerated approval to Merck Pembrolizumab (trade name: Keytruda) for the treatment of patients with unresectable or metastatic melanoma. On May 27, 2017, the FDA approved Keytruda for the treatment of microsatellite unstable solid tumors, becoming the first antibody drug to treat a variety of cancers. Pembrolizumab is IgG4, which have a weak affinity for FcR receptors and lack the ability to activate complement.

Based on this, if the antibody constant region can be modified without affecting the affinity and specificity of antibodies, and the ADCC/CDC function of antibodies can be reduced, at the same time, the formation of a large number of polyclonal antibodies can be avoided, and the immunogenicity of antibodies can be reduced, then the safety of monoclonal antibodies can be improved on the one hand. On the other hand, it can increase the half-life of the drug, reduce the dosage and improve the curative effect.

CTLA-4, also known as CD152, is a transmembrane protein encoded by the CTLA-4 gene, expressed in activated CD4+ and CD8+ T cells, and highly homologous to the T-cell-specific surface glycoprotein (CD28) on the T cell surface. Both CTLA-4 and CD28 are members of the immunoglobulin superfamily and bind to the same ligands CD86 (B7-2) and CD80 (B7-1). The key to the immunoregulatory function of CTLA-4 is the control of CD4+FoxP3-, CD8+T cells and regulatory T cells (Treg). CTLA-4 can halt the activated T cell response and mediate the inhibitory of Treg. Current studies have shown that CTLA-4 inhibits T cell response mainly by two ways: first, by competitive binding to B7 with CD28 or by recruiting phosphatase into the intracellular domain of CTLA-4, thus reducing the signal of TCR (T cell receptor) and CD28. The other is to decrease the expression level of CD80 and CD86 in antigen presenting cells (APC), or to remove CD80 and CD86 from APC through transendocytosis, which reduces the involvement of CD28 in T cell activation. In addition, CTLA-4 can also mediate the binding of CD80/CD86 to dendritic cells and induce the expression of tryptophan-degrading enzyme IDO, thus leading to the inhibition of TCR. CTLA-4 antibodies bind to CTLA-4 to reduce Treg cells and activate TCR.

On April 2011, the FDA approved Ipilimumab (trade name: Yervoy) of Bristol-Myers Squibb (BMS) for the treatment of advanced melanoma, making it the first immune checkpoint inhibitor on the market. However, due to its high immune related adverse effects, Ipilimumab has limited therapeutic fields and small market share. PD-1/PD-L1 related antibody drugs occupied much larger market shares. Compared with the annual sales of USD 35 billion of PD-1/PD-L1 related drugs, the annual sales of Ipilimumab antibody drugs was only USD 1.2 billion in 2017. So far, PD-1 and PD-L1 related antibodies approved indications include advanced melanoma, Squamous Cell Carcinoma of the Head and Neck (SCCHN), non-small cell lung cancer (NSCLC), urothelial carcinoma, non-hodgkin's lymphoma, Merkel's cell carcinoma and microsatellite instability (MSI-H) solid tumor, meanwhile indications for ipilimumab were limited to unresectable or metastatic melanoma. In addition, compared with PD-1 antibody, ipilimumab is less effective in treating advanced melanoma and has a higher incidence of immune-related side effects. This brings risks and inconvenience to clinical patients, and greatly limits the market shares and sales of this antibody drug.

Ipilimumab was IgG1 subtype, which has strong ADCC/CDC activity. To a certain extent, such strong ADCC effect will lead to immunotherapy induced side effects, which are mainly generated by FcR-mediated specific elimination of Treg cells in the tumor microenvironment. Therefore, a De-ADCC/CDC effect antibody will effectively reduce the incidence of ADCC associated side effects.

The ipilimumab of BMS was anti-CTLA-4 human IgG1 subtype antibody with a dose of 3mg/kg, administered intravenously every 3 weeks for a total of four doses. The side effects of ipilimumab are very strong, and 10-20% of patients treated with ipilimumab alone have severe adverse reactions or even death due to the over-activation of T cells. Combined with Opdivo, the anti-tumor effect increased significantly, however, more than half of patients experienced grade 3-4 severe side effects. Some patients even permanently discontinue medication for severe adverse reactions. It was found that ipilimumab not only temporarily stop the suppression of the immune system by blocking the CTLA-4 signaling pathway, but also killed the T-regulating cells due to its ADCC activity, which once and for all removed the patient's immunosuppression regulation, leading to excessive activation of the immune system. If ipilimumab can be modified by removing the ADCC/CDC activity while retaining the neutralization effect of the original drug, the immunotoxicity related to immunotherapy will be greatly reduced, and the incidence of grade 3-4 severe side effects will be significantly reduced, which makes the therapeutic antibody much safer.

We present a novel method to reduce ADCC, CDC and other Fc function(s) by the insertion of amino acid sequence in the Fc of antibody drug.

### SUMMARY OF THE INVENTION

The first purpose of the present invention is to provide a method for reducing the ADCC/CDC and other Fc functions of the antibody and its applications.

The second purpose of the invention is to provide a series of therapeutic antibodies with De-ADCC/CDC activity, including a variety of monoclonal antibodies against PD-L1, PD-1, CTLA-4, etc., and their applications.

For the purpose of the invention, the following technical approach is adopted.

The present invention provides a method for reducing or removing the ADCC/CDC and other Fc-mediated activities of the IgG1 subtype antibody by inserting a flexible amino acid sequence in the constant region of the IgG1 antibody's heavy chain.

In some specific examples of the present invention, the method inserts a flexible amino acid sequence between the CDR3 and CH2 regions of the heavy chain of the original IgG1 subtype antibody.

Described further, the insertion of the flexible amino acid sequence can block the mechanical stress transmit from antibody's variable region to its constant region after the binding to antigen. The Fc and/or complement binding sites in heavy chain of the antibody will not be sufficiently exposed to Fc receptor and/or complement without this stress transmission. The insertion of the flexible sequence can weaken or block the interaction between antibody and NK cells, macrophages or neutrophils, which expressing IgG Fc receptors or complement, leading to the reduced or even eliminated ADCC and CDC activities.

In some specific implementation schemes of the present invention, the modified antibodies can be anti-PD-Ll, anti-PD-1, anti-CTLA-4, anti-TNF-α, anti-PCSK9, anti-NGF, anti-C5, anti-Aβ, anti-IL-6, anti-IL-17A, anti-IL23A, anti-HGF, anti-CMET, anti-Notchl, anti-CCLll, anti-IL6R, anti-IL-31R, anti-IL-lB, anti-IL-20, anti-CD40, anti-CD47, anti-DKK1/2, anti-TIGIT, anti-4-1BB, anti-IGF-lR, anti-LL4, anti-PDGFR2, anti-HER3, anti-IGF1/2, anti-RANKL, anti-sclerostin, anti-GCGR, anti-CGRP, anti-ANGPTL3, anti-IL-13, anti-IL-4R, anti-CSF-lR, anti-TFPI, anti-FCGRT, anti-CD47 antibodies, such as Atezolizumab, Pembrolizumab, Ipilimumab, Eculizumab, evolocumab, erenumab, fulranumab, crenezumab, brodalumab, ixekizumab, satralizumab, gevokizumab, denosumab, blosozumab, crotedumab, fasinumab, fremanezumab, evinacumab, lebrikizumab, dupilumab, cabiralizumab, concizumab, rozanolixizumab, magrolimab, etc., the subtype of which is IgG1.

In some specific implementation schemes of the present invention, the flexible amino acid sequence includes but is not limited to GGSGGS, GSGGSGG, GSGGSGGG, GGGGSGGG, GSGSG, GGSGG, GGS, GGSGS, and GGGS.

In some specific implementation schemes of the present invention, the flexible amino acid sequence insertion sites include but are not limited to GG (138/139), SS (177/178), SG (178/179), SS (184/185), SSS (191/192/193), LL (235/236), GG (237/238).

The invention also provides an application of a flexible amino acid sequence in improving the stability of the antibody/reducing the immunogenicity of the antibody/prolongs the half-life of the antibody.

In some specific implementation schemes of the present invention, the flexible amino acid sequence is inserted into the constant region of the IgG1 antibody heavy chain.

In some specific implementation schemes of the present invention, the flexible amino acid sequence is inserted between CDR3 and CH2 of the IgG1 antibody heavy chain.

The present invention also provides a class of therapeutic antibodies with low ADCC/CDC, low content of the polymer and low immunogenicity, including anti-PD-L1 antibody.

The present invention also provides a method for generating monoclonal antibodies of low ADCC/CDC, low content of the polymer and low immunogenicity therapeutic antibodies including anti-PD-L1 antibody.

The present invention is based on the mechanism of antibody induced ADCC/CDC, by inserting a flexible amino acid sequence in the constant region of the antibody's heavy chain, the mechanical stress transmission resulting from the binding antigen in the variable region of the antibody is interrupted, so that the binding site in the antibody drug heavy chain constant region is not fully exposed to the Fc receptor and/or the complement. The insertion of the flexible sequence can weaken the interaction between antibody and NK cells, macrophages or neutrophils, which expressing IgG Fc receptors or complement, leading to the reduced or even eliminated ADCC and CDC activity.

The present invention is also based on the restoration of glycosylation by mutating 297 amino acid A back to N of Genentech's Atezolizumab, and then a flexible amino acid sequence was inserted between the heavy chain CDR3 and CH2 regions of IgG1 antibody to block the stress transmission between the variable region and constant region of the antibody after binding the antigen, so as to reduce the ADCC/CDC activity while not significantly increasing the formation of the polymers.

The low immunogenicity anti-PD-L1 monoclonal antibody of the present invention is to modify the amino acid in the non-antigen-binding region of the original Atezolizumab sequence to reduce its immunogenicity. The amino acid sequences of the full length of the heavy chain and the variable region of the light chain of the original Atezolizumab sequence are shown in SEQ ID NO.1 and 2 respectively.

Further, the low immunogenicity anti-PD-L1 monoclonal antibody provided by the invention has the amino acid sequence of SEQ ID NO.3 or 4 for its heavy chain and SEQ ID NO.2 for its light chain.

The light chain of the anti-human PD-L1 monoclonal antibody provided by the invention is as shown in L0 (SEQ ID NO.2), and the full length of the heavy chain has any sequence as shown in the two sequences of H-1 (SEQ ID NO.3) and H-2 (SEQ ID NO.4).

With these light chain variable region and heavy chain variable region, the present invention screening for two monoclonal antibody sequences, they not only maintain the original binding affinity to human PD-L1, but also can specifically block the binding of PD-1 to PD-L1 and reduce the content of polymers, leading effectively reduction of their immunogenicity. The ADCC activity verification results show that the ADCC activity of the two monoclonal antibody sequences screened by the invention is consistent with that of the original Atezolizumab.

The two low immunogenicity monoclonal antibodies against PD-L1 are respectively H-1L0 and H-2L0.

The invention provides the application of the monoclonal antibody or the biological material containing these antibody in the treatment of diseases targeting PD-L1 or PD-1.

The invention provides the application of the monoclonal antibody or the biological material containing the antibody for the purpose of killing tumor cells or treating diseases with degenerative immune capacity.

The biomaterial is an expression kit, an expression vector, an engineered bacteria or a cell.

The invention provides the application of the above two low immunogenicity monoclonal antibodies against PD-L1 in the generation of therapeutic drugs for diseases.

The disease is tumor or diseases with degenerative immune capacity.

The invention provides the application of the above-mentioned anti-PD-L1 monoclonal antibody in the generation of PD-L1 targeted disease therapeutic drugs.

The drugs are anti-tumor and immunodegenerative diseases drugs.

The invention provides a drug or detection reagent containing the above-mentioned anti-PD-L1 monoclonal antibody.

The present invention provides the application of the De-ADCC/CDC functional anti-PD-L1 monoclonal antibody in the treatment of diseases targeting PD-L1 or PD-1.

The present invention provides the application of the De-ADCC/CDC functional anti-PD-L1 monoclonal antibody in killing tumor cells or treating diseases with degenerative immune capacity.

The anti-PD-Ll monoclonal antibody of the invention can be combined with a second therapeutic agent or therapeutic form. Anti-PD-L1 antibodies may be combined with cancer therapy that includes the administration of either CTLA-4 or CD47 antibodies. Examples of non-restrictive combinations include anti-PD-L1 antibodies in combination with anti-CTLA-4 antibodies for the treatment of melanoma or non-small cell lung cancer.

The treatment with the anti-PD-Ll antibody of the present invention can be combined with chemotherapy. Chemotherapy such as cytotoxic agents causes the apoptosis of the cancer cells, thereby increasing the release of tumor antigens. The increased effectiveness of tumor antigens can lead to synergistic effects with anti-PD-L1 antibody therapy.

The PD-L1 monoclonal antibody of the present invention can be combined with surgery to remove cancer cells from the treated patients.

The anti-PD-L1 antibody of the present invention can be combined with the therapy that is synergistic with the blocking of PD-L1, the therapies include targeted drugs for hormonal elimination or inhibition of angiogenesis, or drugs that target proteins that are active in tumor cells. All of these lead to increased tumor cell death and increased effectiveness of immunostimulatory tumor antigens.

The anti-PD-L1 monoclonal antibody of the present invention can be combined with another therapeutic antibody for the treatment of cancer or infectious diseases. Specific examples can be: the combination of anti-PD-L1 antibody and the antibody targeting PD-1 or CTLA-4.

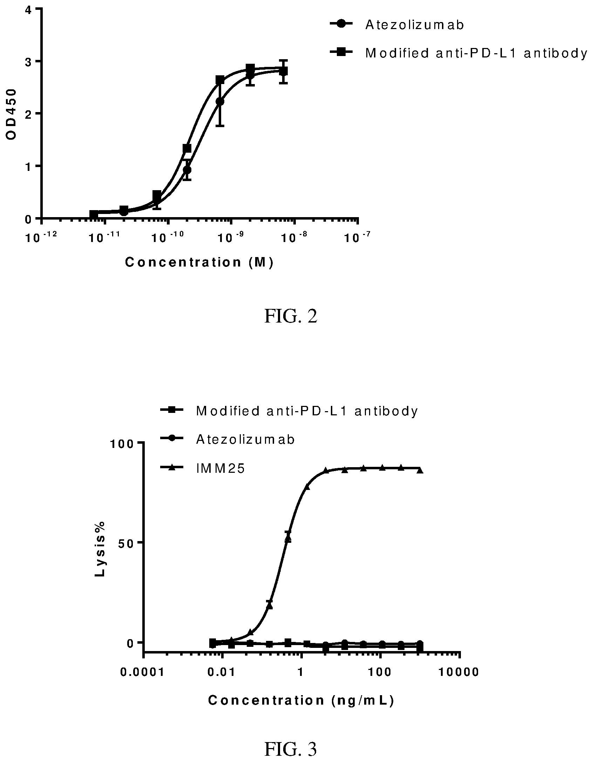

The invention provides a method for generation of the above-mentioned anti-PD-L1 monoclonal antibody, which includes the following steps:
(1) The invention used Pymol to analyze the structure of Atezolizumab of Genentech. The relatively flexible region was found between the variable region and the constant region of the antibody, and the flexible amino acid sequence was inserted into the corresponding region, and the optimized sequence was synthesized and sequenced.
(2) According to the known information of the heavy chain glycosylation of the antibody, the invention synthesizes the corresponding modified sequence. Sequencing the synthetic gene and select the correct one for the next step. The variable region of the light chain was designed with the restriction enzyme cutting site Kpn I + BamH I, while the variable region of the heavy chain was designed with the restriction enzyme cutting site Kpn I+Age I. The synthetic gene was connected to the expression vector pJH16 and transformed into E. *coli* DH5α.
(3) Plasmid extraction was carried out for the above expression vectors, and the Qiagen endotoxin-free plasmid extraction kit was selected.
(4) The plasmid of different heavy and light chain was randomly combined, and CHO cells were used for transient transfection expression. Binding affinity and EC50 value were detected for the expressed antibody (see Figure 2). According to the results, the best combination was selected for the construction of the stable cell line.
(5) According to the above results, the invention selects the corresponding combination to construct the stable cell line by electrotransfection, and MTX was used for screening. Several stable cell lines were obtained after multiple pressure screening, and the stable strain with high expression yield was finally selected for subsequent verification.
(6) The immune response of monoclonal antibody produced by the present invention in mice is lower than that of Atezolizumab.

The anti-PD-L1 antibody provided by the invention targets at the same site of PD-L1 as Atezolizumab, but its immunogenicity and conformation are different from that of Atezolizumab. Compared with Atezolizumab, its stability is significantly improved, immunogenicity is greatly reduced, and the half-life of drugs in animals is prolonged, which is expected to be an ideal antibody for biological targeted therapy. By modifying Atezolizumab monoclonal antibody sequence, the invention will significantly reduce the risk of the antibody drug producing immunogenicity in the patient. The embodiment of the present invention shows that the binding affinity of the modified anti-PD-L1 antibody to PD-L1 is similar to that of the original Atezolizumab. The modification significantly reduces ADA titer in animals and prolongs the half-life of the antibody drug, and improves the curative effect.

The present invention also provides the IgG1 subtype De-ADCC/CDC activity monoclonal antibody against human PD-1 and the application of the monoclonal antibody.

The present invention is based on the mechanism of antibody induced ADCC/CDC, change the antibody from IgG4 subtype to IgG1 subtypeby, then inserting a flexible amino acid sequence in the constant region of the antibody's heavy chain, the mechanical stress transmission resulting from the binding antigen in the variable region of the antibody is interrupted, so that the binding site in the antibody drug heavy chain constant region is not fully exposed to the Fc receptor and/or the complement. The insertion of the flexible sequence can weaken the interaction between antibody and NK cells, macrophages or neutrophils, which expressing IgG Fc receptors or complement, leading to the reduced or even eliminated ADCC and CDC activity. The full length amino acid sequence of the original Pembrolizumab heavy chain is shown in SEQ ID NO.5, and the full length amino acid sequence of the original Pembrolizumab light chain is shown in SEQ ID NO.6.

Further, the present invention provides a IgG1 subtype De-ADCC/CDC activity PD-1 monoclonal antibody, the full length of its heavy chain contains the amino acid sequence shown in SEQ ID NO.7 or the variant of the amino acid sequence shown in SEQ ID NO.7, the full length of the light chain contains the amino acid sequence as shown in SEQ ID No.6 or a variant of the amino acid sequence as shown in SEQ ID No.6.

In any of the above embodiments, the constant region of the antibody heavy chain or its variant is IgG1.

The antibody of the present invention may include: Insert a flexible amino acid sequence between the heavy chain CDR3 and CH2 regions of IgG1 antibody. And the mechanical stress transmission between the variable region and constant region resulting from the binding antigen in the variable region of the antibody is blocked, which can reduce the ADCC/CDC activity as well as the difficulty of development of antibody production process.

Specifically, the present invention provides a IgG1 subtype De-ADCC/CDC activity anti-PD-1 monoclonal antibody. The full length amino acid sequence of the light chain is shown in L0 (SEQ ID NO.6), and the full length amino acid sequence of the heavy chain is shown in H1 (SEQ ID NO.7).

The H1L0 monoclonal antibody sequence is obtained by screening, which was the combination of the above-mentioned variable regions of light chain and heavy chain. It not only maintains the original binding affinity to human PD-1, which can specifically block the binding of PD-1 and PD-L1, but also reduces the activity of ADCC and CDC. And as it is IgG1 subtype antibody, the production process is mature and requires less effort than the original Pembrolizumab IgG4 subtype antibody, and the anti-aggregation ability, expression level and stability of the antibody are improved.

The present invention provides an expression vector containing the genes of any of the IgG1 subtypes with De-ADCC/CDC activity PD-1 monoclonal antibody light chain and heavy chain. The host bacteria, host cells or expression boxes containing the expression vector are also within the protection scope of the invention.

The present invention provides a method for increasing the activity of immune cells by giving therapeutic effective amounts of antibodies to the object in need of treatment. The method can be used to treat cancer and immunocompromised diseases. The invention also includes further administration of a second therapeutic agent or therapeutic form.

The invention also includes the use of the above-mentioned anti-PD-1 monoclonal antibody in the generation of drugs for improving immune function.

The anti-PD-1 monoclonal antibody of the invention can be combined with a second therapeutic agent or therapeutic form. Anti-PD-1 antibodies may be combined with cancer therapies that include the administration of recombinant cytokines or secreted immune factors. Non-restrictive examples of combinations include anti-PD-1 antibodies in combination with recombinant IL-2 or recombinant IFN2 in the treatment of melanoma or renal cell carcinoma. Recombinant IL-2 increases T cell production in cancer patients. Recombinant IFN2 not only inhibits the growth of cancer cells in treated patients, but also increases the expression of inhibitory ligands of PD-1 on cancer cells, antigen presenting cells and other somatic cells. The anti-PD-1 monoclonal antibody of the present invention may be combined with other cytokines that are thought to be useful in the treatment of cancer or infectious diseases.

The anti-PD-1 monoclonal antibody or antibody fragment of the present invention may be combined with a vaccine to prevent or treat cancer or infectious diseases. As non-restrictive examples, anti-PD-1 monoclonal antibody can be combined with: one or more antigens protein, peptide or DNA vaccine associated with the treatment of cancer or infection or the vaccine consisting of dendritic cells stimulated with the antigens. The invention provides the use of an anti-PD-1 antibodies combine with (weakened) cancer cell or whole virus vaccine. The invention provides a combination of anti-PD-1 therapy and a whole-cell cancer vaccine that is genetically engineered to secret GM-CSF.

The anti-PD-1 monoclonal antibody of the present invention can be combined with a treatment considered to be the standards of care in cancer or infectious diseases. The rationale for the combination is that increased immune activation by anti-PD-1 antibodies will induce or promote an initial clinical response to the standards of care treatment, induce a long-term clinical response, and induce long-term immune control of the disease.

The treatment with the anti-PD-1 antibody of the present invention can be combined with chemotherapy. Chemotherapy such as cytotoxic agents causes the apoptosis of the cancer cells, thereby increasing the release of tumor antigens. The increased effectiveness of tumor antigens can lead to synergistic effects with anti-PD-1 therapy.

The PD-1 monoclonal antibody of the present invention can be combined with surgery to remove cancer cells from the treated patients.

The anti-PD-1 antibody of the present invention can be combined with a therapy that can produce a synergistic effect with the blocking of PD-1. The therapies include targeted drugs for hormonal elimination or inhibition of angiogenesis, or targeting proteins active in tumor cells. All of these lead to increased tumor cell apoptosis and increased effectiveness of immunostimulatory tumor antigens. In combination with anti-PD-1 antibodies, increased T cell activation leads to sustained immune control of cancer.

The anti-PD-1 monoclonal antibody of the present invention can be combined with another therapeutic antibody for the treatment of cancer or infectious diseases. Specific examples include: combination of anti-PD-1 antibody and antibody targeting Her2/neu or EGF receptor; combination of anti-PD-1 antibody and anti-CTLA-4 antibodies; combination of anti-PD-1 antibody or antibody fragment and antibody targeting OX40.

The invention provides the aforesaid monoclonal antibody or the biological material containing the monoclonal antibody in the treatment of diseases targeting PD-L1 or PD-1.

The invention provides the monoclonal antibody or the biomaterial containing the monoclonal antibody for the purpose of killing tumor cells or treating diseases with degenerative immune capacity.

The biomaterial is an expression kit, an expression vector, an engineered bacteria or a cell.

The present invention provides the application of IgG1 subtype De-ADCC/CDC activity anti-PD-1 monoclonal antibody in the generation of drugs to treat diseases or improve immune function.

The disease is tumor or diseases with degenerative immune capacity.

The present invention provides the application of IgG1 subtype De-ADCC/CDC activity PD-1 monoclonal antibody in the generation of PD-L1 targeted therapeutic drugs for diseases.

The disease is tumor or diseases with degenerative immune capacity.

The present invention provides a drug or detection reagent containing the IgG1 subtype De-ADCC/CDC activity PD-1 monoclonal antibody.

The present invention provides the application of IgG1 subtype De-ADCC/CDC activity PD-1 monoclonal antibody in the treatment of diseases targeting PD-L1 or PD-1.

The present invention provides the application of IgG1 subtype De-ADCC/CDC activity PD-1 monoclonal antibody in the killing of tumor cells or in the treatment of immunodegenerative diseases.

The invention used Pymol to analyze the structure of Pembrolizumab of Merck (USA), switch the antibody from IgG4 subtype to IgG1 subtype. A flexible amino acid sequence was inserted between CDR3 and CH2 regions of the heavy chain of IgG1 antibodies, which can reduce the activity of ADCC/CDC and the difficulty of subsequent production process of antibodies, improve the anti-aggregation ability, expression level and stability of antibodies, and avoid the formation of semi-antibodies and bispecific antibodies caused by the heavy chain replacement characteristics of IgG4 subtypes. The monoclonal antibody has the same binding affinity to human PD-1 as the original Pembrolizumab, and can specifically block the binding of PD-1 to cell surface PD-L1, prolong the half-life of the antibody drug, and improve the curative effect. Therefore, it has excellent clinical application value.

The present invention also provides the IgG1 subtype De-ADCC/CDC activity monoclonal antibody against human CTLA-4 and the application of the monoclonal antibody.

The present invention is based on the mechanism of antibody induced ADCC/CDC. A flexible amino acid sequence was inserted between CDR3 and CH2 regions of the heavy chain of IgG1 antibodies, so that the binding site in the antibody drug heavy chain constant region is not fully exposed to the Fc receptor and/or the complement, which can weaken the interaction between antibody and NK cells, macrophages or neutrophils, which expressing IgG Fc receptors or complement, leading to the reduced or even eliminated ADCC and CDC activity. The full-length amino acid sequence of the Ipilimumab heavy chain is shown as SEQ ID NO.8, and the full length amino acid sequence of the Ipilimumab light chain is shown as SEQ ID NO.9.

The invention used Pymol to analyze the structure of Ipilimumab of Bristol-Myers Squibb (BMS). A flexible amino acid sequence was inserted between CDR3 and CH2 regions of the heavy chain of IgG1 antibodies, which can reduce the activity of ADCC/CDC and the side effects of antibodies in treatment, improve the anti-aggregation ability, expression level and stability of antibodies. The binding affinity of the monoclonal antibody to human CTLA-4 is similar to that of the original Ipilimumab, and the monoclonal antibody can specifically block the binding of CTLA-4 to CD80 and CD86, reduce the side effects of antibody drug treatment and improve the curative effect of antibody drug, which has excellent clinical application value.

Further, the present invention provides a IgG1 subtype De-ADCC/CDC activity CTLA-4 monoclonal antibody. The full length of its heavy chain contains the amino acid sequence as shown in SEQ ID No.10, and the full length of its light chain contains the amino acid sequence as shown in SEQ ID No.9.

In any of the above embodiments, the heavy chain subtype of the antibody is IgG1.

The antibody of the invention may contain: A flexible amino acid sequence was inserted between CDR3 and CH2 regions of the heavy chain of IgG1 antibodies to block the stress transmission between the variable region and constant region of the antibody after binding the antigen, which can reduce the activity of ADCC/CDC and the difficulty of subsequent production process of antibodies, improve the anti-aggregation ability, expression level and stability of antibodies.

Specifically, IgG1 subtype De-ADCC/CDC activity CTLA-4 monoclonal antibody is provided. The full length light chain amino acid sequence is shown as L0 (SEQ ID NO.9), and the full length heavy chain amino acid sequence is shown as H1 (SEQ ID NO.10).

The invention screened anti-CTLA-4 antibody H1L0. It not only maintains the binding affinity to human CTLA-4, but can specifically block the binding of CTLA-4 to its ligand, and reduce the activity of ADCC and CDC.

The present invention provides an expression vector containing any genes of the IgG1 subtypes with De-ADCC/CDC activity CTLA-4 monoclonal antibody light chain and heavy chain. The host bacteria, host cells or expression boxes containing the expression vector are also within the protection scope of the invention.

The present invention provides a method for increasing the activity of immune cells by giving therapeutic effective amounts of antibodies to the patients in need of treatment. The method can be used to treat cancer and immunocompromised diseases. The invention also includes further administration of a second therapeutic agent or therapeutic form.

The invention also includes the use of the above-mentioned anti-CTLA-4 monoclonal antibody in the generation of drugs for improving immune function.

The anti-CTLA-4 monoclonal antibody of the present invention can be combined with a second therapeutic agent or therapeutic form. Anti-CTLA-4 antibody can be combined with cancer therapy that includes the administration of either anti-PD-1 or anti-PD-L1 antibody. Non-restrictive examples of combinations include anti-CTLA-4 antibody in combination with anti-PD-1 antibody in the treatment of melanoma or non-small cell lung cancer.

The treatment with the anti-CTLA-4 antibody of the present invention can be combined with chemotherapy. Chemotherapy such as cytotoxic agents causes the apoptosis of the cancer cells, thereby increasing the release of tumor antigens. The increased effectiveness of tumor antigens may lead to synergistic effects with anti-CTLA-4 therapy.

The anti-CTLA-4 monoclonal antibody of the present invention can be combined with surgery to remove cancer cells from the treated patients.

Anti-CTLA-4 antibody of the present invention can be combined with the therapy that works synergistically with CTLA-4 blocking. The therapies include targeted drugs for hormonal elimination or inhibition of angiogenesis, or targeting proteins active in tumor cells. All of these lead to increased tumor cell apoptosis and increased effectiveness of immunostimulatory tumor antigens. In combination with anti-CTLA-4 antibodies, increased T cell activation leads to sustained immune control of cancer.

The anti-CTLA-4 monoclonal antibody of the present invention can be combined with another therapeutic antibody for the treatment of cancer or infectious diseases. Specific examples can be: the combination of anti-CTLA-4 antibody and anti-PD-1 or anti-PD-L1 antibody.

The application of the monoclonal antibody or the biomaterial containing the monoclonal antibody provided in the present invention in the treatment of diseases targeting CTLA-4.

The invention provides the monoclonal antibody or the biomaterial containing the monoclonal antibody for the purpose of killing tumor cells or treating diseases with degenerative immune capacity.

The biomaterial is an expression kit, an expression vector, an engineered bacteria or a cell.

The present invention provides the application of IgG1 subtype De-ADCC/CDC activity anti-CTLA-4 monoclonal antibody in the generation of drugs to treat diseases or improve immune function.

The disease is tumor or diseases with degenerative immune capacity.

The present invention provides the application of the IgG1 subtype De-ADCC/CDC activity anti-CTLA-4 monoclonal antibody in the generation of CTLA-4-targeted therapeutic drugs for diseases.

The disease is tumor or diseases with degenerative immune capacity.

The present invention provides the combined application of IgG1 subtype De-ADCC/CDC activity anti-CTLA-4 monoclonal antibody in the treatment of diseases targeting PD-L1 or PD-1.

The present invention provides the application of the IgG1 subtype De-ADCC/CDC activity anti-CTLA-4 monoclonal antibody in the killing of tumor cells or in the treatment of immunodegenerative diseases.

To sum up, the method of reducing the ADCC/CDC function of the antibody of the present invention can reduce the ADCC/CDC activity while not significantly increasing the formation of polymers. The modified monoclonal antibody and the original monoclonal antibody target the same binding epitope, but the conformation of the antibodies is different. For the modified antibodies, the stability is significantly improved, the immunogenicity is significantly reduced, and the half-life is prolonged. Effectively avoid the side effects caused by ADCC/CDC activity, improve the curative effect, and have excellent clinical application value.

### BRIEF DESCRIPTION OF THE DRAWINGS

FIG. 1 is the heavy chain sequence alignment of the modified PD-L1 monoclonal antibody (H-1L0 and H-2L0) and the original Atezolizumab.
FIG. 2 is the binding affinity of modified PD-L1 monoclonal antibody and the original Atezolizumab. The x-coordinate is the antibody concentration (M), and the y-coordinate is the absorbance at 450nm.
FIG. 3 is the ADCC activity evaluation of modified PD-L1 monoclonal antibody (H-1L0 and H-2L0) and the original Atezolizumab measured by an *in vitro* cell killing assay using PD-L1 overexpressing Raji cells together with FcR overexpressing TANK cells in the presence or absence of PD-L1 antibodies. The x-coordinate is the sample concentration (ng/mL), and the y-coordinate is the percentage of lysed raji-PDLl cells, the IMM25 is an ADCC-enhanced anti-PD-Ll antibody used as the positive control, and the original Atezolizumab was used as the control for ADCC evaluation.
FIG. 4 is the inhibitory activity of modified PD-L1 monoclonal antibody and the original Atezolizumab measured by *in vitro* cell-based Bioassay using Jurkat-PD-1-NFAT cell line that stably expresses PD-1 with the reporter luciferase gene. The x-coordinate is antibody concentration and the y-coordinate is mean value of the relative fluorescence units (RLU).
FIG. 5 is the ADA titers evaluation from the mice immunized with either modified PD-L1 monoclonal antibody or the original Atezolizumab measured using Indirect ELISA against antibody drugs. The x-coordinate is the serum dilution ratio and the y-coordinate is the absorbance value at 450nm.
FIGS. 6A and 6B are graphs of the original Atezolizumab and the modified PD-L1 monoclonal antibody measured by HPLC-SEC.
FIGS. 7A and 7B are the glycon type analysis of the original Atezolizumab and the modified PD-L1 monoclonal antibody of the invention.
FIG. 8 is the thermal stability evaluation of the original Atezolizumab and the modified PD-L1 monoclonal antibody of the invention. The x-coordinate is the antibody concentration (M), and the y-coordinate is the absorbance at 450nm. The H-1L0 stands for modified anti-PD-Ll antibody.
FIG. 9 is the heavy chain sequence alignment of the modified PD-1 monoclonal antibody and the original Pembrolizumab.
FIG. 10 is the binding affinity of modified PD-1 monoclonal antibody and the original Pembrolizumab. The x-coordinate is the antibody concentration (M), and the y-coordinate is the absorbance at 450nm.
FIG. 11 is the ADCC activity evaluation of modified PD-1 monoclonal antibody and the original Pembrolizumab measured by an *in vitro* cell killing assay using PD-1 overexpressing Raji cells together with FcR overexpressing TANK cells in the presence or absence of PD-1 antibodies. The x-coordinate is the sample concentration (ng/mL), and the y-coordinate is the percentage of lysed raji-PDl cells. Raji cell express CD20 on its membrane, so the Rituximab, which is an anti-CD20 antibody, can be used as positive control for ADCC evaluation.
FIG. 12 is the inhibitory activity of modified PD-1 monoclonal antibody and the original Pembrolizumab measured by *in vitro* cell-based Bioassay using Jurkat-PD-1-NFAT cell line that stably expresses PD-1 with the reporter luciferase gene. The x-coordinate is antibody concentration and the y-coordinate is the inhibition rate.
FIG. 13 is the heavy chain sequence alignment of the modified CTLA-4 monoclonal antibody and the original Ipilimumab.
FIG. 14 is the binding affinity of modified CTLA-4 monoclonal antibody and the original Ipilimumab. The x-coordinate is the antibody concentration (M), and the y-coordinate is the absorbance at 450nm.
FIG. 15 is the ADCC activity evaluation of modified CTLA-4 monoclonal antibody and the original Ipilimumab measured by an *in vitro* cell killing assay using CTLA-4 overexpressing Raji cells together with FcR overexpressing TANK cells in the presence or absence of CTLA-4 antibodies. The x-coordinate is the sample concentration (ng/mL), and the y-coordinate is the percentage of lysed raji-PD1 cells.
FIG. 16 is the inhibitory activity of modified CTLA-4 monoclonal antibody and the original Ipilimumab measured by ELISA-based Bioassay. The x-coordinate is antibody concentration (M) and the y-coordinate is the absorbance value at 450nm. The H1L0 stands for modified anti-CTLA-4 antibody.
FIG. 17 is the competition of antibodies with CD80 and CD86 to bind to human CTLA-4 expressed CHO cells. The x-coordinate is antibody concentration (M) and the y-coordinate is the absorbance value at 450nm. The H1L0 stands for modified anti-CTLA-4 antibody.

### DETAILED DESCRIPTION OF THE PREFERRED EMBODIMENT

All references mentioned herein are specifically incorporated by reference.

### Abbreviations and Definitions

"Antibody" means any form of an antibody that exhibits the biological activity required (for example, by inhibiting ligand binding to its receptor or by inhibiting ligand-induced receptor signaling). Therefore, the antibodies are used in the broadest sense and explicitly include, but are not limited to, monoclonal antibodies, polyclonal antibodies, and poly-specific antibodies.

The "Fab fragment" consists of the CH1 region of a light chain and a heavy chain as well as the variable region. The heavy chain of one Fab molecule cannot form the disulfide bond with that of another molecule.

The "Fc" region is the two heavy chain fragments containing the CH1 and CH2 domains of the antibodies. The two heavy chain fragments are held together by two or more disulfide bonds and by hydrophobic interaction in the CH3 domain.

The "Fab' fragment" contains a light chain and a heavy chain that contains the VH domain, the CH1 domain and the region between CH1 and CH2 domains, thus an interchain disulfide bond can be formed between the two heavy chains of the two Fab' fragments to form the F(ab')2 molecule.

The term "monoclonal antibody" used in this application refers to antibodies derived from essentially homologous antibody groups, that is, the antibodies that make up the group are consistent except for possible natural mutations that may be present in small quantities. Monoclonal antibodies are highly specific and can target a single antigen site. Furthermore, in contrast to the conventional (polyclonal) antibody generation, which usually consists of several different antibodies against several different determinants (epitopes), each monoclonal antibody targets only a single determinant on an antigen. The term "monoclonal" denotes the characteristics of an antibody obtained from a substantially homoclonal antibody group and cannot be understood as requiring the generation of the antibody by any particular method.

The term "flexible amino acid sequence" used in this application refers to a segment of connecting polypeptide consisting of amino acids with smaller side chains, which is used to prevent or reduce the interference on the structure conformation between the structural domains at its two ends, including but not limited to GGSGGS, GSGGSGG, GSGGSGGG, GGGGSGGG, GSGSG, GGSGG, GGS, GGSGS and GGGS.

The term "immune cell" used in this application includes cells that have a hematopoietic origin and play a role in the immune response. Immune cells include: lymphocytes, such as B lymphocytes and T lymphocytes; Natural killer cells; Myeloid cells, such as monocytes, macrophages, eosinophilic cells, mast cells, basophils, and granulocytes.

The term "approximate" used in this application means that the index value is within the acceptable error range of the specific value measured by the general technician in this field, and that the value depends in part on how it is measured (that is, the limitation of the measurement system). For example, in each practice in this field, "about" may mean a standard deviation within or above 1. Alternatively, "about" or "substantially included" can imply a range of up to 20 per cent. In addition, especially for biological systems or processes, the term may imply at most one order of magnitude or at most five times the value. Unless otherwise stated, the meaning of "about" or "substantially contained" when a particular value appears in this application and claim shall be assumed to be within an acceptable error range for that particular value.

When referring to ligands/receptors, antibodies/antigens, or other binding pairs, "specific" binding refers to the presence of the binding reaction of the mentioned protein, such as PD-1, in a heterogeneous population of proteins and/or other biological reagents. Thus, under specified conditions, specific ligands/antigens bind to specific receptors/antibodies and do not significantly bind to other proteins present in the sample.

When the terms "to give" and "to treat" refer to an animal, a person, an experimental subject, a cell, tissue, organ or biological fluid, it means to bring an exogenous drug, therapeutic agent, diagnostic agent or composition into contact with an animal, a person, a treated subject, a cell, tissue, organ or biological fluid. "To give" and "to treat" may mean, for example, therapeutic methods, pharmacokinetic methods, diagnostic methods, research methods and experimental methods. The treatment of cells consists of contacting the reagents with the cells and the reagents with the fluids, wherein the fluids are in contact with the cells. "To give" and "to treat" also imply, for example, in vitro treatment of cells by means of reagents, diagnostics, binding compositions or other cells.

"Effective dose" includes an amount sufficient to ameliorate or prevent the symptoms or symptoms of a medical disease. Effective dose also refer to quantities sufficient to enable or facilitate diagnosis. The effective dose available to a specific patient may vary depending on a number of factors, such as the disease to be treated, the patient's overall health status, the approach and dose administered, and the severity of side effects. The effective dose may be the maximum dose or regimen administered to avoid significant side effects or toxicity. Monoclonal antibody generation, this invention relates to antibody can be put the DNA in expression vectors, transfection the vectors into the host cell then implement the synthesis of the monoclonal antibodies in a recombinant host cell. The host cells can be E*.coli* cells, Chinese hamster ovary (CHO) cells or myeloma cells that do not produce additional immunoglobulin. The generation of recombinant antibodies is described in more detail below.

Appropriate routes of administration include parenteral (e.g., intramuscular, intraenous, or subcutaneous) and oral. Antibodies of the present invention for use in pharmaceutical compositions or in the practice can be administered in a variety of conventional ways, such as oral ingestion, inhalation, topical application, or injection through the skin, subcutaneous, intraperitoneal, parenteral, intraarterial, or intravenous. Antibodies can be administered locally rather than in a systemic way (usually in the form of long-acting formulations or sustained-release formulations), for example by injecting antibodies directly into the site of action. In addition, antibodies can be given in targeted drug delivery systems.

The term "inhibit", "treat" or "treatment" used in this invention includes delaying the development of symptoms associated with the disease and/or reducing the severity of these symptoms that the disease will or is expected to develop. The terms also include mitigating existing symptoms, preventing additional symptoms, and mitigating or preventing the underlying causes of those symptoms. Thus, the term implies that beneficial results have been assigned to vertebrate objects suffering from disease.

The term "therapeutic efficency dose" or "efficency dose" used in this article refers to the amount of antibody or its fragment that is effective in preventing or mitigating a disease or condition to be treated when given alone or in combination with another therapeutic agent to cells, tissues or persons being treated. Therapeutic effective dose further refers to the amount of the compound sufficient to cause relief of symptoms, such as to treat, cure, prevent or mitigate the related medical condition, or to increase the rate of treatment, cure, prevention or mitigation of the symptoms. When an individual is given a single active ingredient, the therapeutic efficency dose refers to that single ingredient. When applied in combination, the therapeutic efficency dose is the amount of the combination of the active ingredients that produced the therapeutic effect, regardless of whether they were given in combination, continuously or simultaneously. The therapeutic effective dose will usually reduce symptoms by at least 10%; Usually at least 20%; Preferably at least about 30%; Preferred at least 40% and preferred at least 50%.

The pharmaceutical composition of the present invention may also contain other agents, including but not limited to cytotoxic agents, cell growth inhibitors, antiangiogenic or antimetabolic drugs, tumor-targeting drugs, immunostimulants or immune modulators, or antibodies combined with cytotoxic agents, cell production inhibitors, or other toxic drug conjugates. The drug composition may also be administered in conjunction with other forms of treatment, such as surgery, chemotherapy and radiation therapy.

Typical veterinarians, experiments, or research subjects include monkeys, dogs, cats, rats, mice, guinea pigs, rabbits, horses, and humans.

The therapeutic application of the antibody of the invention is as follows:
Antibodies of the present invention specifically bind to human PD-1/L1 and CTLA4 and can be used to increase, improve, stimulate or up-regulate the immune response. Antibodies of the present invention are particularly suitable for treating patients suffering from diseases that can be treated by increasing the T-cell-mediated immune response. Preferred recipients include patients who need to improve their immune response.

For cancer, the antibody of the present invention can be used to treat cancer (i.e., inhibit the growth or survival of tumor cells). Preferred cancers for which antibodies of the present invention can inhibit their growth include those that normally respond to immunotherapy, but also those that have not hitherto been associated with immunotherapy. Optimizing for the treatment of cancer of unrestricted examples include melanoma (such as malignant metastatic melanoma), kidney cancer (such as clear cell carcinoma), prostate cancer (such as hormone-refractory prostate cancer, HRPC) and pancreatic carcinoma, breast cancer, colorectal carcinoma, lung cancer (non-small cell lung cancer, for example), esophageal carcinoma, head and neck squamous carcinoma, liver cancer, ovarian cancer, cervical cancer, thyroid cancer, glioblastoma, glioma, leukemia, lymphoma and other malignant tumors. In addition, the present invention includes refractory or recurrent cancers whose growth can be inhibited by the antibody of the present invention.

The antibody can be used alone or in combination with the following substances: anti-tumor drugs or immunogenics (such as weakened cancer cells), tumor antigens (including recombinant proteins, peptides, and glycogens), antigen-presenting cells such as dendritic cells stimulated by antigens or nucleic acids derived from tumors, immune-stimulating cytokines (e.g. Il-2, IFNa2, GM-CSF and cells transfected with genes encoding immune-stimulating cytokines (such as, but not limited to, GM-CSF)); standard cancer treatment (such as chemotherapy, radiation or surgery); or other antibodies (including, but not limited to, antibodies against: VEGF, EGFR, Her2/neu, VEGF receptor, other growth factor receptor, CD20, CD40, CTLA-4, OX-40, 4-IBB, and ICOS).

For immunocompromised diseases, the antibody of the present invention can also be used to prevent or treat immunocompromised diseases. Antibodies can be used alone or in combination with drugs to stimulate immune responses to pathogens or polymers. Antibodies can be used to stimulate an immune response to viruses that infect humans, such as, but not limited to, human immunodeficiency virus, hepatitis virus (A, B, or C), Epstein-Barr virus, human cytomegalovirus, human papilloma virus, and herpes virus. Antibodies can be used to stimulate an immune response to infections caused by bacterial or fungal parasites and other pathogens. Antibodies can be used to stimulate the immune response and remove the polymers in vivo, and treat alzheimer's disease and other diseases.

The antibody of the invention to the target includes but is not limited to PD-L1, PD-1, CTLA4, TNF-α, PCSK9, NGF, C5, Aβ, IL-6, IL-17A, IL23A, HGF, CMET, Notch1, CCL11, IL6R, IL-31R, IL-1B, IL-20, CD40, CD47, DKK1/2, TIGIT, 4-1BB, IGF-1R, LL4, PDGFR2, HER3, IGF1/2, RANKL, sclerostin, GCGR, CGRP, ANGPTL3, IL-13, IL-4R, CSF-1R, TFPI, FCGRT, CD47, etc., which can be used to treat cancer, asthma, autoimmune diseases, migraine, Alzheimer's disease, hyperlipidemia, osteoarthritis, cluster headache, osteoporosis and other diseases.

The following examples further state the contents of the present invention, but shall not be construed as the limitation of the present invention. Without deviating from the spirit and substance of the invention, any modification or substitution of the method, procedure or condition of the invention falls within the scope of the invention.

If not specifically specified, the technical methods used in examples are conventional methods familiar to the technician in the field. Materials, reagents, etc., used in the following examples can be obtained commercially without special instructions.

### EXAMPLE 1 Analysis and design of the low immunogenic anti-PD-Ll monoclonal antibody with De-ADCC/CDC activity

The invention used Pymol to analyze the structure of Atezolizumab of Genentech. The relatively flexible region was found between the variable region and the constant region of the antibody, and the flexible amino acid sequence was inserted into the corresponding region. The insertion of the flexible amino acid sequence can block the mechanical stress transmit from variable area to constant region after the combining of antigen and antibody. The Fc and/or complement binding sites in heavy chain of the antibody is not sufficiently exposed to Fc receptor and/or complement without this stress transmission. The insertion of the flexible sequence can weaken the interaction between antibody and NK cells, macrophages or neutrophils, which expressing IgG Fc receptors or complement, leading to the reduced or even eliminated ADCC and CDC activity.

Specifically, the present invention is based on the restoration of glycosylation by mutating 297 amino acid A back to N of Genentech's Atezolizumab, and then a flexible amino acid sequence was inserted between the heavy chain CDR3 and CH2 regions of IgG1 antibody to block the stress transmission between the variable region and constant region of the antibody after binding the antigen, so as to reduce the ADCC/CDC activity while not significantly increasing the formation of the polymers. The amino acid sequences of the original Atezolizumab sequence with full length of heavy chain and variable region of light chain are shown in SEQ ID NO.1 and 2, respectively.

The low immunogenicity anti-PD-L1 monoclonal antibody provided by the invention has the heavy chain amino acid sequence of SEQ ID NO.3 and 4, and the light chain amino acid sequence of SEQ ID NO.2, respectively named H-1L0 and H-2L0.

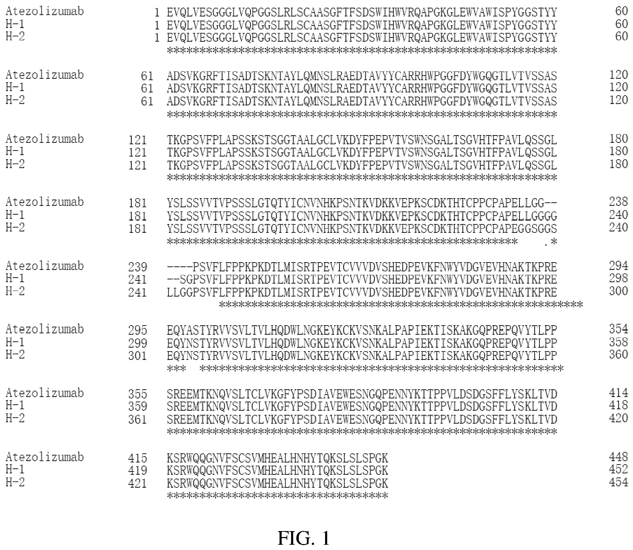

According to the known information of the glycosylation of the antibody heavy chain, the invention synthesizes the corresponding modified sequence, sequenced the synthesized gene and selects the correct sequence for the next operation. The restriction site was designed as EcoR I + Hind III for both the heavy chain and the light chain. Then the sequences were connected to the expression vectors pEE12.4 and pEE6.4 for heavy chain and light chain respectively. E. *coli* DH5 was transformed to obtain the expression vector of heavy chain and light chain of antibody. Meanwhile, sequencing and sequence alignment were proceeded for the constructed antibodies. The sequence alignment results of the modified anti-PD-L1 monoclonal antibody with the original Atezolizumab heavy chain were shown in Figure 1.

### EXAMPLE 2 Analysis and design of the anti-PD-1 monoclonal antibody with De-ADCC/CDC activity

The invention modified the Pembrolizumab of Merck (USA). First, switch the antibody from IgG4 subtype to IgG1 subtype. Then, analyze the structure of the Pembrolizumab with Pymol, the relatively flexible region was found between the variable region and the constant region of the antibody, and the flexible amino acid sequence was inserted into the corresponding region. The invention synthesizes the corresponding modified sequence, sequenced the synthesized gene and selects the correct sequence for the next operation. Monoclonal antibody (H1L0) was generated.

Specifically, the present invention inserted a flexible amino acid sequence between the heavy chain CDR3 and CH2 regions of IgG1 antibody to block the stress transmission between the variable region and constant region of the antibody after binding the antigen. The Fc and/or complement binding sites in heavy chain of the antibody is not sufficiently exposed to Fc receptor and/or complement without this stress transmission. So the insertion of the flexible sequence can weaken the interaction between antibody and NK cells, macrophages or neutrophils, which expressing IgG Fc receptors or complement, leading to the reduced or even eliminated ADCC and CDC activity. Flexible amino acid sequence was the polypeptide consisting of amino acids with smaller side chains, including but not limited to GGSGGS, GSGGSGG, GSGGSGGG, GGGGSGGG, GSGSG, GGSGG, GGS, GGSGS and GGGS, to prevent or reduce interference in conformation between different domains. The modified heavy chain amino acid sequence according to the method described in the present invention is shown as SEQ ID No.7.

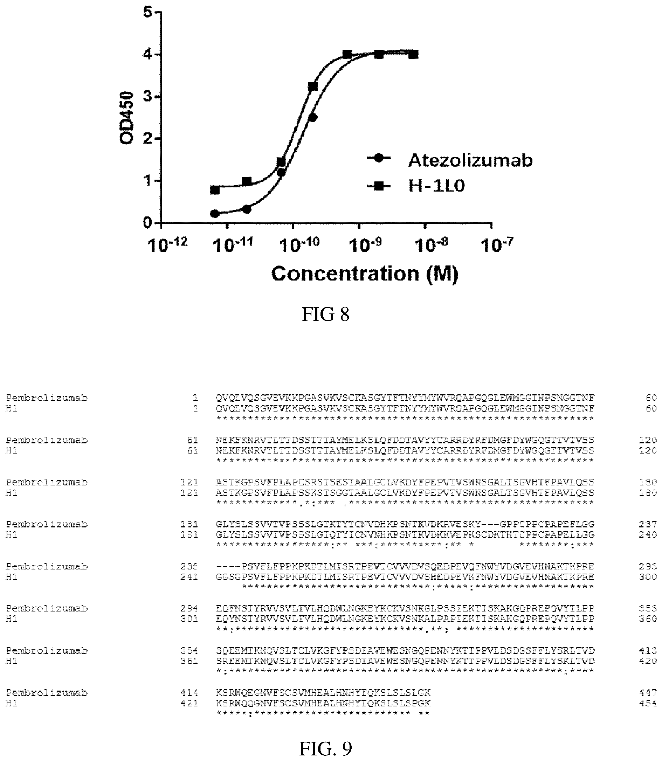

The invention synthesizes the corresponding modified sequence, sequenced the synthesized gene and selects the correct sequence for the next operation. The restriction site was designed as EcoR I + Hind III for both the heavy chain and the light chain. Then the sequences were connected to the expression vectors pEE12.4 and pEE6.4 for heavy chain and light chain respectively. E. *coli* DH5 was transformed to obtain the expression vector of heavy chain and light chain of antibody. Meanwhile, sequencing and sequence alignment were proceeded for the constructed antibodies. The sequence alignment results of the modified anti-PD-1 monoclonal antibody with the original Pembrolizumab heavy chain were shown in Figure 9.

### EXAMPLE 3 Analysis and design of the anti-CTLA-4 monoclonal antibody with De-ADCC/CDC activity

The invention modified the Ipilimumab of Bristol-Myers Squibb (BMS). Analyze the structure of the Ipilimumab with Pymol, the relatively flexible region was found between the variable region and the constant region of the antibody, and the flexible amino acid sequence was inserted into the corresponding region. The invention synthesizes the corresponding modified sequence, sequenced the synthesized gene and selects the correct sequence for the next operation. Monoclonal antibody (H1L0) was generated. Specifically, the present invention inserted a flexible amino acid sequence between the heavy chain CDR3 and CH2 regions of IgG1 antibody to block the stress transmission between the variable region and constant region of the antibody after binding the antigen. The Fc and/or complement binding sites in heavy chain of the antibody is not sufficiently exposed to Fc receptor and/or complement without this stress transmission. So the insertion of the flexible sequence can weaken the interaction between antibody and NK cells, macrophages or neutrophils, which expressing IgG Fc receptors or complement, leading to the reduced or even eliminated ADCC and CDC activity. Flexible amino acid sequence was the polypeptide consisting of amino acids with smaller side chains, including but not limited to GGSGGS, GSGGSGG, GSGGSGGG, GGGGSGGG, GSGSG, GGSGG, GGS, GGSGS and GGGS, to prevent or reduce interference in conformation between different domains. The modified heavy chain amino acid sequence according to the method described in the present invention is shown as SEQ ID No.10.

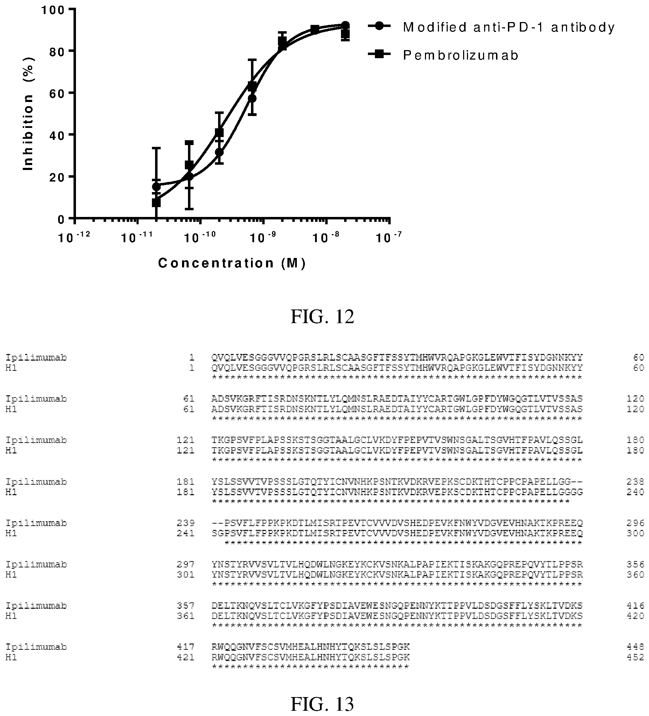

The invention synthesizes the corresponding modified sequence, sequenced the synthesized gene and selects the correct sequence for the next operation. The restriction site was designed as EcoR I + Hind III for both the heavy chain and the light chain. Then the sequences were connected to the expression vectors pEE12.4 and pEE6.4 for heavy chain and light chain respectively. E. *coli* DH5 was transformed to obtain the expression vector of heavy chain and light chain of antibody. Meanwhile, sequencing and sequence alignment were proceeded for the constructed antibodies. The sequence alignment results of the modified anti-CTLA-4 monoclonal antibody with the original Ipilimumab heavy chain were shown in Figure 13.

### EXAMPLE 4 ADCC activity verification of modified antibody

The ADCC activity of the antibody is evaluated by detecting the killing ability of the FcR-TANK cells to the overexpressed cells of target proteins (PD-L1, PD-1 and CTLA-4) in the presence of the relevant antibodies.

Take the ADCC activity detection of anti-PD-L1 antibody as an example: Using CFSE to dye Raji cells overexpressing target protein (such as PD-L1, PD-1 or CTLA-4), then adjust the Raji and FcR-TANK cell density to 6×10⁵/mL respectively. The modified antibody, Atezolizumab and the positive control antibody (Purchased from ImmuneOnco Biopharma) are diluted with a proportional gradient (the highest concentration is 4 µg/mL and triply diluted to 12 gradients with medium).

Incubate the antibodies, target cells and effector cells together: 50 µL of antibodies with different dilution gradients, 50 µL of target cells and 100 µL of FcR-TANK cells were added to the 96-well plate. Parallel wells were performed for each gradient and two different blank controls (culture medium 150 µL+ target cells 50 µL and culture medium 50 µL+ target cells 50 µL+ FcR-TANK cells 100 µL) were set. Incubate at 37°C, with 5% CO₂ for 4 hours. At the end of incubation, place the plate at room temperature for 10min, then 5µg/mL PI was added to stain the dying Raji cells. And the cell populations were analyzed using flow-cytometer. For calculation of ADCC activity: ADCC% = [(Sample % PI Positive cell - Control % PI Positive cell) / (100 - Control % PI Positive cell)] × 100. The relation curve between ADCC% and concentration is drawn.

The ADCC activity evaluation results showed the modified anti-PD-L1 monoclonal antibody of the present invention has the same low/no ADCC activity as the original Atezolizumab, as shown in Figure 3.

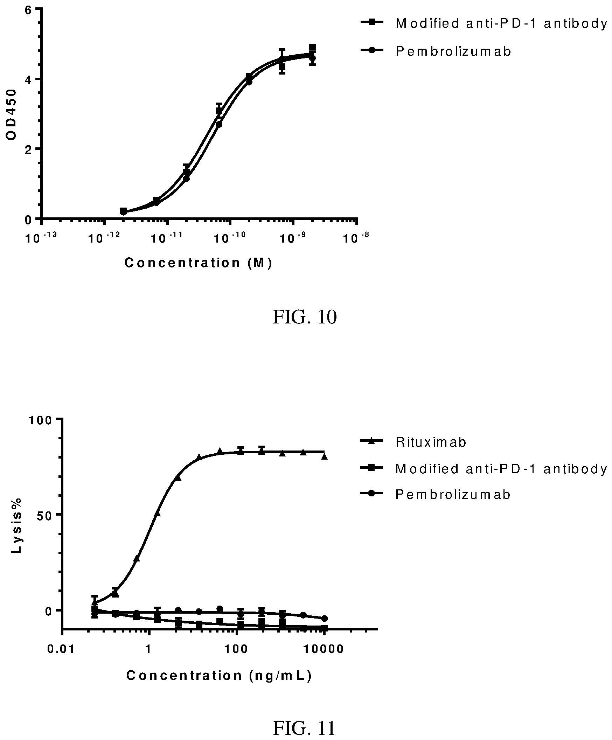

The ADCC activity evaluation results showed that the modified PD-1 monoclonal antibody lost its ADCC activity, while the original Pembrolizumab showed relatively weak ADCC activity, as shown in Figure 11.

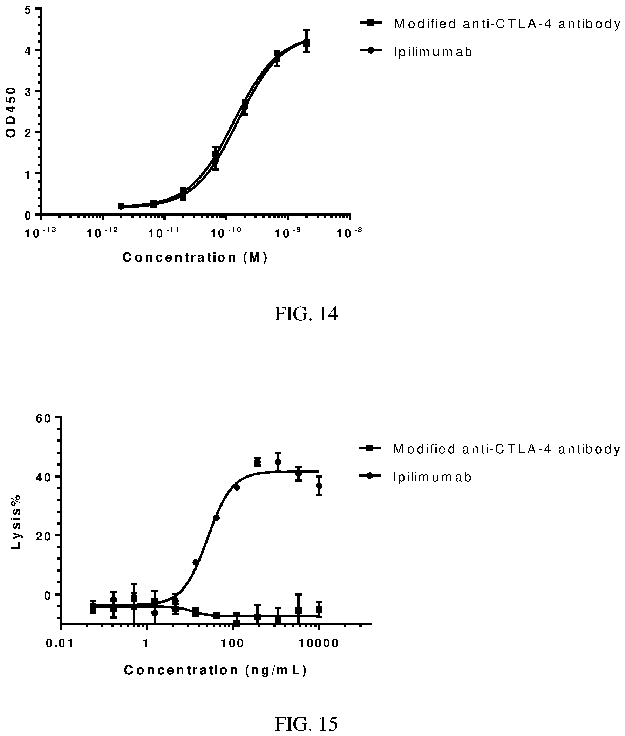

The ADCC activity evaluation results showed that the ADCC activity of anti-CTLA-4 monoclonal antibody was lost, while the ADCC activity of Ipilimumab was normal, as shown in Figure 15.

### EXAMPLE 5 Affinity evaluation of modified antibodies

Plasmid extraction was carried out for the vector constructed in EXAMPLE 1-3 with Qiagen endotoxin-free plasmid extraction kit. The plasmids of different heavy and light chains were randomly combined, and CHO cells were used for transient transfection expression. Protein-based ELISA was used to determine the binding affinity (EC50 value was reported), which can be used to compare the binding ability between the modified and original antibodies.

ELISA was used to determine the relative binding affinity of antibodies to human PD-L1. PD-L1 was immobilized on the plate at 4°C overnight, and the non-specific binding site was blocked by incubation with 3% BSA-PBS at room temperature for 1 hour. After coating, the plate was washed with PBS and the diluent of anti-PD-L1 antibodies was prepared, which was incubated with the immobilized protein at 25°C for 1 hour. After the incubation, the plate was washed with PBS for 3 times, and incubated at 25°C with binding buffer containing peroxidase-labeled Goat anti-human IgG diluted to 1/2000 for 1 hour. and then washed three times again and add TMB for color development. The ELISA results are shown in Figure 2. The magnitude of the relative binding affinity is expressed by the semi-maximum binding concentration. Affinity and EC50 were detected for the antibodies (see Table 1). The results showed that the affinity of modified anti-PD-L1 antibody and PD-L1 was similar to that of the original Atezolizumab (see Figure 2).

**Table 1 Evaluation of antibody binding affinity**

| mAb | EC₅₀ (pM) |
|---|---|
| Atezolizumab | 320.3 |
| Modified anti-PD-L1 antibody | 219.4 |

ELISA was used to determine the relative binding affinity of antibodies to human PD-1. PD-1 was immobilized on the plate at 4°C overnight, and the non-specific binding site was blocked by incubation with 3% BSA-PBS at room temperature for 1 hour. After coating, the plate was washed with PBS and the diluent of anti-PD-1 antibodies was prepared, which was incubated with the immobilized protein at 25°C for 1 hour. After the incubation, the plate was washed with PBS for 3 times, and incubated at 25°C with binding buffer containing peroxidase-labeled Goat anti-human IgG diluted to 1/2000 for 1 hour. and then washed three times again and add TMB for color development. The ELISA results are shown in Figure 10. The magnitude of the relative binding affinity is expressed by the semi-maximum binding concentration. Affinity and EC50 were detected for the antibodies (see Table 2). The results showed that the affinity of modified anti-PD-1 antibody and PD-1 was similar to that of the original Atezolizumab (see Figure 10).

**Table 2 Evaluation of antibody binding affinity**

| mAb | EC₅₀ (pM) |
|---|---|
| Pembrolizumab | 54.5 |
| Modified anti-PD-1 antibody | 43.7 |

ELISA was used to determine the relative binding affinity of antibodies to human CTLA-4. CTLA-4 was immobilized on the plate at 4°C overnight, and the non-specific binding site was blocked by incubation with 3% BSA-PBS at room temperature for 1 hour. After coating, the plate was washed with PBS and the diluent of anti-CTLA-4 antibodies was prepared, which was incubated with the immobilized protein at 25°C for 1 hour. After the incubation, the plate was washed with PBS for 3 times, and incubated at 25°C with binding buffer containing peroxidase-labeled Goat anti-human IgG diluted to 1/2000 for 1 hour. and then washed three times again and add TMB for color development. The ELISA results are shown in Figure 14. The magnitude of the relative binding affinity is expressed by the semi-maximum binding concentration. Affinity and EC50 were detected for the antibodies (see Table 3). The results showed that the affinity of modified anti-CTLA-4 antibody and CTLA-4 was similar to that of the original Atezolizumab (see Figure 14).

**Table 3 Evaluation of antibody binding affinity**

| mAb | EC₅₀ (pM) |
|---|---|
| Ipilimumab | 155.0 |
| Modified anti-CTLA-4 antibody | 134.9 |

### EXAMPLE 6 Bioactivity evaluation of modified anti-PD-1 and anti-PD-L1 antibodies

This method is based on two cell lines, CHO-PD-L1-CD3L cell line and Jurkat-PD-1-NFAT cell line. CHO-PD-L1-CD3L cell line stably expressed PD-L1 and anti-CD3-scFv, which can be uesd as target cells; The Jurkat-PD-1-NFAT cell line stably expressed PD-1 and luciferase (the luciferase gene was regulated by NFAT elements (transcription factors) (IL-2 promoter)), which can be used as effector cells. The binding of PD-1 and PD-L1 can block the downstream signal transduction of CD3, thus inhibiting the expression of luciferase. When PD-1 antibody or PD-L1 antibody is added, this block effect is reversed, and luciferase can be expressed, thus detecting the fluorescence signal.

The specific experimental procedures are as follows:
1. Cell culture: Jurkat-PD-1-NFAT cells were cultured in RPMI1640 medium containing 10%FBS, 1%NEAA and antibiotics. CHO-PD-L1-CD3L cells were cultured in DMEM or F12 medium containing 10%FBS, 1%NEAA and antibiotics.
2. Cell inoculation: CHO-PD-L1-CD3L cells were seeded in 96-well plates with 50000 cells per well and cultured for 12-14 hours at 37°C and 5% CO₂. After the removing of the medium, 50 µL analytical medium (RPMI1640 medium containing 2%FBS) with 100,000 Jurkat-PD-1-NFAT cells were added into each well.
3. Antibody screening: The modified antibody or the original antibody is diluted in a gradient ratio of 1:3 from a concentration of 10 µg/mL (using the analytical medium). The diluted antibodies were then added to each well and incubated at 37°C with 5% CO2 for 6 hours.
4. Add 100 µL Promega Bio-Glotm Luciferase Assay substrate. The relative luciferase activity units were evaluated by the SpectraMax M5.

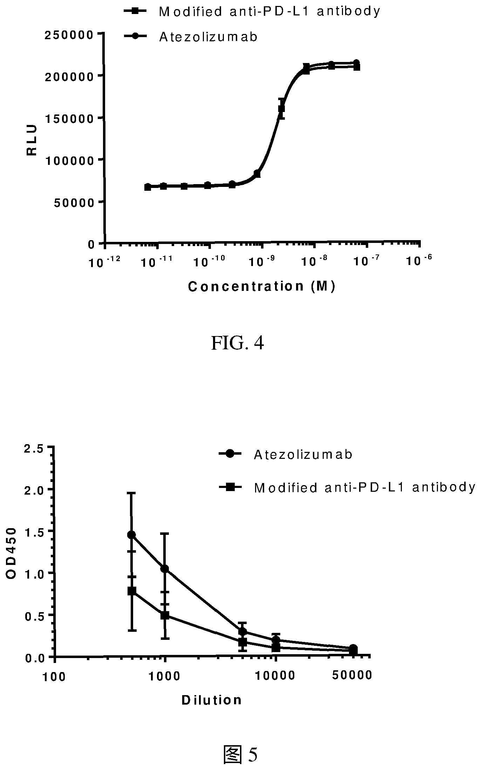

The results show that the modified anti-PD-Ll antibody and the original Atezolizumab have similar inhibitory effects on PD-1/PD-L1 (As shown in Figure 4). The diagram was drawn and the IC50 values of antibodies against the interaction of PD-1 and PD-L1 are calculated, as shown in Table 4.

**Table 4 Evaluation of antibody inhibitory effect**

| mAb | IC₅₀ (nM) |
|---|---|
| Atezolizumab | 1.988 |
| Modified anti-PD-Ll antibody | 1.945 |

The results show that the modified anti-PD-1 antibody and the original Pembrolizumab have similar inhibitory effects on PD-1/PD-L1 (As shown in Figure 12). The diagram was drawn and the IC50 values of antibodies against the interaction of PD-1 and PD-L1 are calculated, as shown in Table 5.

**Table 5 Evaluation of antibody inhibitory effect**

| mAb | IC₅₀ (nM) |
|---|---|
| Pembrolizumab | 1.986 |
| Modified anti-PD-1 antibody | 1.943 |

### EXAMPLE 7 The modified anti-CTLA-4 antibody inhibits the binding of CTLA-4 to CD80 or CD86

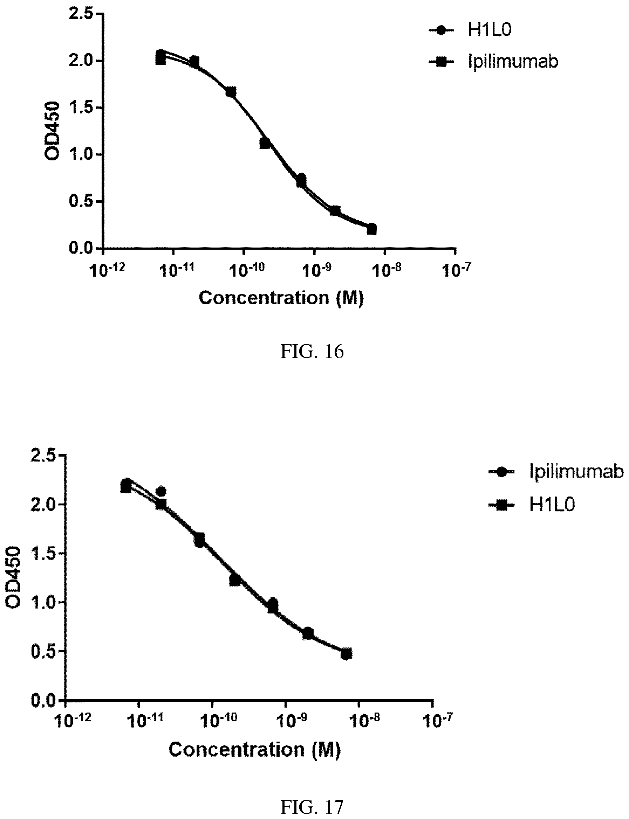

ELISA was used to detect the binding efficiency of CTLA-4 with CD80 or CD86. hCD80 or hCD86 were immobilized on the plate at 4°C overnight, and the non-specific binding sites were blocked by incubation with 3%BSA-PBS at room temperature for 1 hour. After coating, the plate was washed with PBS and the mixed diluent of anti-CTLA-4 antibody and Biotin-CTLA-4 (biotin-CTLA-4 was a fixed concentration of 1 µg/mL, and the anti-CTLA-4 antibody is gradient diluted with the highest concentration of 1 µg/mL and the lowest concentration of 0 µg/mL) was prepared. The mixed diluent was incubated with the immobilized protein at 25°C for 1 hour. After the incubation, the plate was washed with PBS for 3 times, and incubated at 25°C with binding buffer containing peroxidase-labeled Avidin diluted to 1/8000 for 1 hour. And then washed three times again and add TMB for color development. As shown in Figure 16, anti-CTLA-4 monoclonal antibody of the present invention inhibit the binding of CTLA-4 to CD80 or CD86.

The CELISA method is used to evaluate the efficacy of the antibody in competition with CD80 and CD86 binding to human CTLA-4 transfected CHO cells. CHO cells expressing hCTLA-4 were first constructed, add 50 µL per well and cultured to 80%-100% coverage of CHO-hCTLA-4 cells in the well. Then, 50 µL mixed diluent containing fixed concentration (1 µg/mL) biotin-CD80 or Biotin-CD86 and different dilution concentrations (the highest concentration is 1 µg/mL, the lowest concentration is 0 µg/mL, gradient dilution) of anti-CTLA-4 antibody was added and incubated at 37°C for 1 hour. After the incubation, the plate was washed with PBS for 3 times, and incubated at 25°C with binding buffer containing peroxidase-labeled Avidin diluted to 1/8000 for 1 hour. And then washed three times again and add TMB for color development. The ELISA results are shown in Figure 17.

### EXAMPLE 8 Immunogenicity evaluation of modified anti-PD-Ll antibody and Atezolizumab in mice

The titer of ADA was evaluated with the serum of modified antibody or Atezolizumab immunized mice by ELISA, respectively. Balb/c mice were used for the experiment with 5 mice in each group. Specific immune procedures were as follows: 1) Basal immunity: the antigen was mixed with The Complete Freud's Adjuvant by the same volume, fully emulsified, and subcutaneously injected at different position; 2) Enhanced immunity: The emulsion of antigens and Incomplete Freud's Adjuvant was used to enhance immunity. The total dose per Balb/c mouse was 70 µg.

Serum was collected from mice 0 days, 7 days, 14 days and 21 days after immunization to evaluate the titer of ADA, the results was shown in Figure 5. The results shown that the ADA titer of monoclonal antibody produced by the invention in mice is 80% lower than that of Atezolizumab.

### EXAMPLE 9 Stable cell line construction and the expression trial of modified anti-PD-Ll antibody

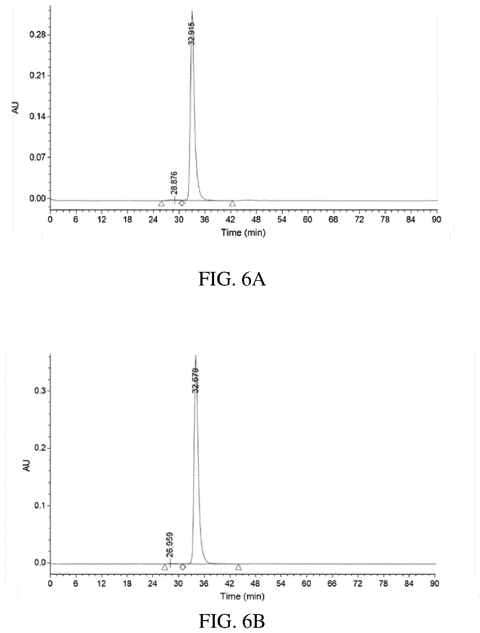

The modified anti-PD-Ll antibody of the invention and the original Atezolizumab were simultaneously constructed with CHO stable cell pool, and 5g of preliminary expression and purification of the modified anti-PD-Ll antibody and the original Atezolizumab were carried out in accordance with the production specifications of antibody drugs known to the public in this field. HPLC-SEC was used to compare the antibodies obtained, as shown in Figure 6A and Figure 6B. The sample loading volume (50 µL) and concentration (60 mg/mL) of Atezolizumab and the modified anti-PD-L1 antibody were the same. It can be seen from the figure that, with the same sample loading volume, the main peak area of Atezolizumab was reduced by nearly 10% compared with that of the modified anti-PD-L1 antibody. Therefore, it is demonstrated that there were more polymers in Atezolizumab. Therefore, during the sample loading of HPLC-SEC, the polymers were removed by the filter, resulting in a decrease in the peak area. The HPLC-SEC peak area calculation results of the two antibodies are shown in Table 6. At the same time, HPLC-SEC results showed that the peak position of the modified anti-PD-L1 antibody was further forward than that of the Atezolizumab, which could to be due to the increase in molecular weight of the antibody caused by the restored glycosylation of the antibody.

**Table 6 HPLC-SEC results of antibodies**

| mAb | | RT | Area | Height | % Area |
|---|---|---|---|---|---|
| Atezolizumab | Polymer | 28.876 | 170858 | 898 | 0.786 |
| | Monomer | 32.915 | 21572737 | 315915 | 99.214 |
| Modified anti-PD-L1 antibody | Polymer | 26.959 | 124831 | 883 | 0.530 |
| | Monomer | 32.679 | 23431856 | 348923 | 99.470 |

### EXAMPLE 10 Glycan isotype evaluation of modified anti-PD-L1 antibodies

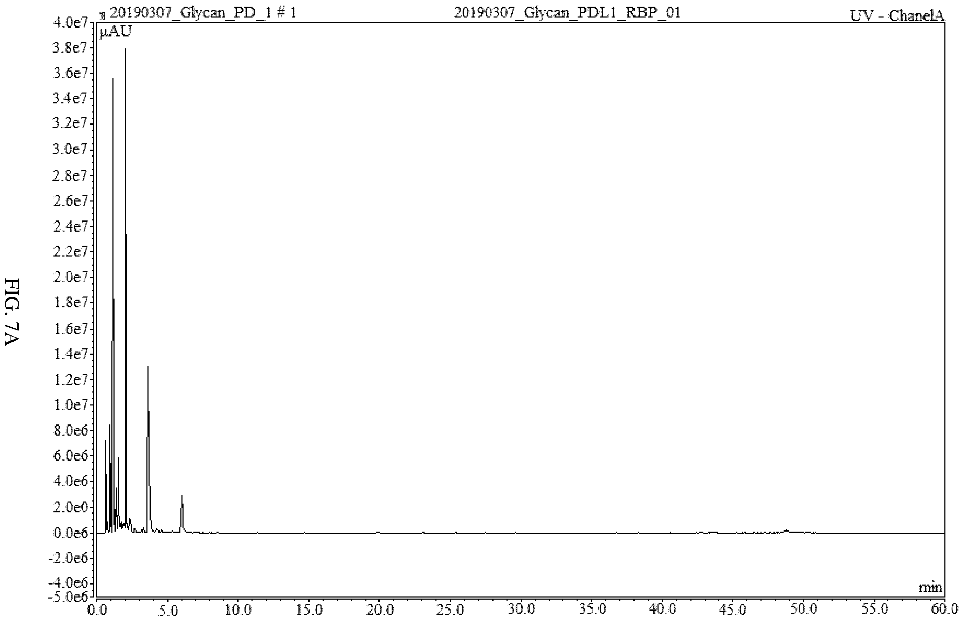

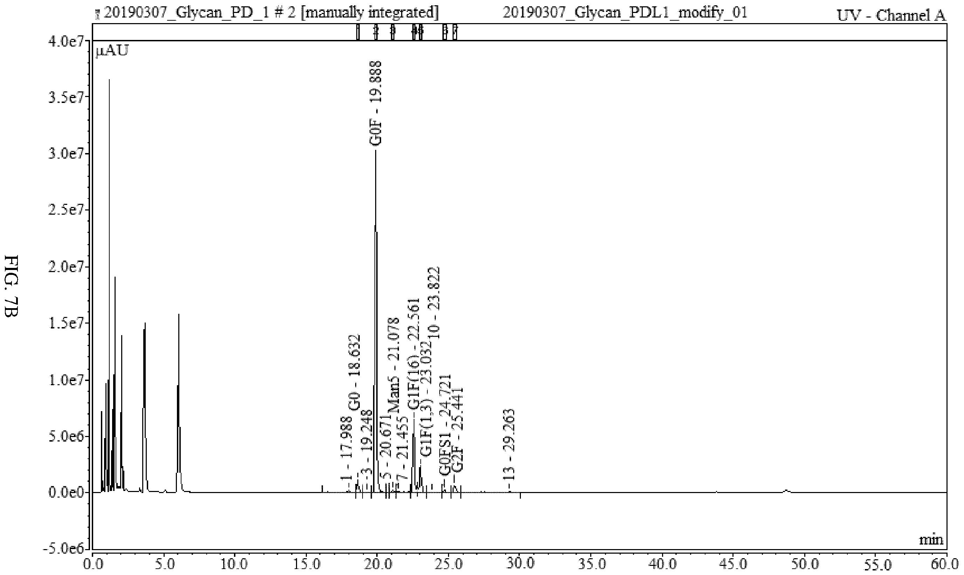

Based on the results of EXAMPLE 9, the modified anti-PD-L1 antibodies was further validated for the glycan isotype. It mainly adopts the method of public knowledge in the field: antibodies were first digested with PNGase F (New England Biolabs) according to the manufacturer's instructions, and the free glycan(s) were separated using LudgerClean™ EB10 kit according to the manufacturer's instructions, and the free glycan(s) were analyzed and identified by mass spectrometry. After verification, the Atezolizumab did not detect the N-glycan modification, while the modified anti-PD-L1 antibody was normal glycosylation modification, as shown in Figure 7. The glycan isotypes of modified anti-PD-Ll antibody are shown in Table 7.

**Table 7 The glycan isotypes of modified anti-PD-L1 antibody**

| | G0 | G0F | Man5 | G1F(1,6) | G1F(1,3) | G0FS1 | G2F |
|---|---|---|---|---|---|---|---|
| relative peak area (%) | 2.52 | 69.80 | 0.82 | 15.10 | 6.24 | 0.57 | 1.76 |

EXAMPLE 11 Stability verification of modified anti-PD-L1 antibody and Atezolizumab The stability of the modified anti-PD-L1 monoclonal antibody and the original Atezolizumab was verified and compared. The modified anti-PD-L1 antibody and the original Atezolizumab were simultaneously subjected to rapid heat treatment, and their stability was verified as follows:
Two antibodies were taken by the same volume and concentration. The samples were taken after standing at 4°C for 10 hours. After the first sampling, the two antibodies were placed at 60°C for 10 minutes. Visually observe the changes in appearance and take samples for the second time. After sampling, the two antibodies were centrifuged at 4°C at 10000rpm for 5 minutes to observe whether there was visible precipitation at the bottom of the centrifuge tube, and the supernatant was sampled again. After the third sampling, the supernatant were filtered by 0.2 µm filter and sampled again. After the last sampling, the supernatant after filtration was placed at 4°C for continuous observation. Antibody concentration and binding affinity of PD-L1 were measured in all samples obtained.

The PD-L1 binding affinity of the modified anti-PD-L1 antibody and the original Atezolizumab were shown in Figure 8, and the concentration of each sample of the two antibodies were shown in Table 8. It can be seen that the modified anti-PD-L1 antibody is more stable than Atezolizumab.

**Table 8 Stability verification of modified anti-PD-L1 antibody and Atezolizumab**

| Sample | Sampling time | Concentration (mg/mL) |
|---|---|---|
| Atezolizumab | Before the heat treatment | 60.8841 |
| | After the heat treatment | 60.3778 |
| | After centrifugation | 56.9252 |
| | After filtration | 49.1294 |
| Modified anti-PD-Ll antibody | Before the heat treatment | 59.8288 |
| | After the heat treatment | 59.3042 |
| | After centrifugation | 57.2363 |
| | After filtration | 57.9317 |

The present invention screened the modified De-ADCC activity monoclonal antibodies against a number of different targets. They not only maintain the binding affinity to the target protein, but also remove its ADCC activity, and can specifically block the binding of the target protein to its corresponding ligand. At the same time, for the modified anti-PD-L1 antibody, by using the ADCC removing method of the invention, the purpose of removing the ADCC effect is not only achieved, but also the problems of high content of polymers and strong immunogenicity caused by the modification of the original antibody drug were solved.

The description of the above examples is intended only to assist in understanding the method and its core ideas of the present invention. It should be noted that for technicians in the field, on the premise of not breaking away from the principle of the present invention, some improvements and modifications may also be made to the present invention, and these improvements and modifications may also fall within the protection scope of the claims of the present invention.

### INDUSTRIAL APPLICABILITY

The present invention provides the De-ADCC/CDC activity of monoclonal antibodies and its generation method and applications. The present invention modified the antibody sequences of multi-target sites such as PD-L1, PD-1, CTLA-4, TNF- α, PCSK9, NGF, CD47 and C5, etc. A flexible amino acid sequence was inserted between the heavy chain CDR3 and CH2 regions of IgG1 antibody to block the stress transmission between the variable region and constant region of the antibody after binding the antigen, so as to reduce the ADCC/CDC and other Fc-mediated activities while not significantly increasing the formation of the polymers. The modified antibodies in the present invention and the original antibodies target the same sites, but the antibody subtype or glycosylation state is different. For the modified antibodies in the present invention, the stability is significantly improved or/and immunogenicity is significantly reduced, and the half-life is prolonged for PD-L1 antibody or others. The antibodies modified by the technology of the present invention eliminated the ADCC/CDC and other Fc-mediated activities activity, therefore it can effectively avoid side effects caused by the activation of ADCC/CDC and improve the curative effect. It has excellent clinical application value, high economic value and broad market prospect, and is suitable for promotion.

## Claims

1. A method of reducing or removing the ADCC/CDC and other Fc-mediated activities of the IgG1 isoform antibody is achieved by the insertion of a flexible amino acid sequence in the constant region of the heavy chain of the IgG1 subtype antibody to stop the stress transmit of conformation change.

2. The method described in Claim 1 is **characterized by** the insertion of a flexible amino acid sequence between the CDR3 and CH2 regions of the heavy chain of the IgG1 subtype antibody to stop the stress transmit of conformation change.

3. Either method as claim 1 or 2 mentioned, its characteristics is described by the insertion of a flexible amino acid sequence, blocking the mechanical stress transmit from variable area to constant region after the combining of antigen and antibody. The Fc and/or complement binding sites in heavy chain of the antibody is not sufficiently exposed to Fc receptor and/or complement without this stress transmission. The insertion of the flexible sequence can weaken or stop the interaction between antibody and NK cells, macrophages or neutrophils, which expressing IgG Fc receptors or complement, leading to the reduced or even eliminated ADCC and CDC activity.

4. Either method as claim 1 or 2 mentioned, its characteristics is the modified antibodies, the subtype of which is IgG1, can be anti-PD-L1, anti-PD-1, anti-CTLA-4, anti-TNF-α, anti-PCSK9, anti-NGF, anti-C5, anti-Aβ, anti-IL-6, anti-IL-17A, anti-IL23A, anti-HGF, anti-CMET, anti-Notchl, anti-CCLll, anti-IL6R, anti-IL-31R, anti-IL-lB, anti-IL-20, anti-CD40, anti-CD47, anti-DKK1/2, anti-TIGIT, anti-4-1BB, anti-IGF-1R, anti-LL4, anti-PDGFR2, anti-HER3, anti-IGF1/2, anti-RANKL, anti-sclerostin, anti-GCGR, anti-CGRP, anti-ANGPTL3, anti-IL-13, anti-IL-4R, anti-CSF-1R, anti-TFPI, anti-FCGRT, anti-CD47 antibodies, such as Atezolizumab, Pembrolizumab, Ipilimumab, Eculizumab, evolocumab, erenumab, fulranumab, crenezumab, brodalumab, ixekizumab, satralizumab, gevokizumab, denosumab, blosozumab, crotedumab, fasinumab, fremanezumab, evinacumab, lebrikizumab, dupilumab, cabiralizumab, concizumab, rozanolixizumab, magrolimab, etc., or the IgG1 subtype modified monoclonal antibody of the antibody drug mentioned above.

5. The method described in Claim 3, its characteristics is the term "flexible amino acid sequence" used in this application refers to a segment of connecting polypeptide consisting of amino acids with smaller side chains, which is used to prevent or reduce the interference on the structure conformation between the structural domains at its two ends, including but not limited to GGSGGS, GSGGSGG, GSGGSGGG, GGGGSGGG, GSGSG, GGSGG, GGS, GGSGS and GGGS.

6. The De-ADCC/CDC monoclonal antibody is **characterized by** the generation of a monoclonal antibody by any of the methods described in claim 1-5.

7. The De-ADCC/CDC activity monoclonal antibodies described in Claim 6 are **characterized by** chimeric monoclonal antibodies, humanized monoclonal antibodies, fully human monoclonal antibodies, bis-specific antibodies, or Fc-fusion proteins.

8. Either of the De-ADCC/CDC activity monoclonal antibodies described in Claim 6-7, its characteristics is the modified antibodies can be anti-PD-L1, anti-PD-1, anti-CTLA-4, anti-TNF-α, anti-PCSK9, anti-NGF, anti-C5, anti-Aβ, anti-IL-6, anti-IL-17A, anti-IL23A, anti-HGF, anti-CMET, anti-Notchl, anti-CCLll, anti-IL6R, anti-IL-31R, anti-IL-1B, anti-IL-20, anti-CD40, anti-CD47, anti-DKK1/2, anti-TIGIT, anti-4-1BB, anti-IGF-1R, anti-LL4, anti-PDGFR2, anti-HER3, anti-IGF1/2, anti-RANKL, anti-sclerostin, anti-GCGR, anti-CGRP, anti-ANGPTL3, anti-IL-13, anti-IL-4R, anti-CSF-1R, anti-TFPI, anti-FCGRT, anti-CD47 monoclonal antibodies.

9. The De-ADCC/CDC activity monoclonal antibodies described in Claim 8 are **characterized by** insertion of a flexible amino acid sequence between the CDR3 and CH2 regions of the heavy chain of the IgG1 subtype antibody. The term "flexible amino acid sequence" used in this application refers to a segment of connecting polypeptide consisting of amino acids with smaller side chains, which is used to prevent or reduce the interference on the structure conformation between the structural domains at its two ends, including but not limited to GGSGGS, GSGGSGG, GSGGSGGG, GGGGSGGG, GSGSG, GGSGG, GGS, GGSGS and GGGS. As well as the application of the method in reducing or eliminating ADCC and CDC activity of IgG1 subtype antibodies.

10. Application of the flexible amino acid sequence in reducing or eliminating the ADCC/CDC activity of IgG1 subtype antibodies.

11. The application described in Claim 10 is **characterized by** the insertion of the flexible amino acid sequence into the constant region of the heavy chain of the IgG1 subtype antibodies.

12. The application described in claim 11 is **characterized by** the insertion of the flexible amino acid sequence between the CDR3 and CH2 region of the heavy chain of the IgG1 antibodies.

13. The application, as described in any of the claims 10-12, is **characterized by** the flexible amino acid sequence insertion sites include but are not limited to GG (138/139), SS (177/178), SG (178/179), SS (184/185), SSS (191/192/193), LL (235/236), GG (237/238).

14. The application described in Claim 13 is **characterized in that** the flexible amino acid sequence includes but is not limited to GGSGGS, GSGGSGG, GSGGSGGG, GGGGSGGG, GSGSG, GGSGG, GGS, GGSGS and GGGS.

15. Application of flexible amino acid sequence to reduce ADCC and CDC activities and other Fc-mediated functions while also improving antibody stability / reducing antibody immunogenicity / prolonging antibody half-life.

16. The application of claim 15, its characteristics is described in the flexible amino acid sequence is inserted into the constant region of IgG1 antibody heavy chain, preferred to described the flexible amino acid sequence is inserted into the heavy chain of IgG1 antibody between CDR3 and CH2 region. The described flexible amino acid sequence includes but not limited to, GGSGGS, GSGGSGG, GSGGSGGG, GGGGSGGG, GSGSG, GGSGG, GGS, GGSGS and GGGS.

17. A De-ADCC/CDC activity anti-PD-L1 monoclonal antibody is **characterized by** the insertion of a flexible amino acid sequence, blocking the mechanical stress transmit from variable area to constant region after the combining of antigen and antibody. The Fc and/or complement binding sites in heavy chain of the antibody is not sufficiently exposed to Fc receptor and/or complement without this stress transmission. The insertion of the flexible sequence can weaken the interaction between antibody and NK cells, macrophages or neutrophils, which expressing IgG Fc receptors or complement, leading to the reduced or even eliminated ADCC and CDC activity.

18. The De-ADCC/CDC activity anti-PD-L1 monoclonal antibodies described in Claim 17 are **characterized by** the insertion of a flexible amino acid sequence between the CDR3 and CH2 region of the heavy chain of IgG1 antibody to block the mechanical stress transmit from variable area to constant region after the combining of antigen and antibody. The amino acid sequences of the heavy chain and the light chain of the original Atezolizumab are shown in SEQ ID NO.1 and 2 respectively.

19. The De-ADCC/CDC activity anti-PD-L1 monoclonal antibodies described in Claim 17 are **characterized by** the full length amino acid sequence of light chain contains as shown in SEQ ID NO.2 or its variants, and the full length amino acid sequence of heavy chain contains any of the sequences as shown in SEQ ID NO.3∼4.

20. Any genes coding De-ADCC/CDC activity anti-PD-L1 monoclonal antibodies as described in claims 17∼19.

21. The biomaterial containing the gene of claim 20, the term "biomaterial" is an expression box, an expression vector, an engineered bacteria or a cell.

22. Any of the following applications of the gene described in Claim 20 or the biomaterial described in claim 21:
(1) Application in the generation of drugs targeting PD-L1 or PD-1;
(2) Application in the treatment of diseases targeted by PD-L1 or PD-1;
(3) Applications in the treatment of tumors or immunodegenerative diseases.

23. Any of the following applications of the De-ADCC/CDC activity anti-PD-Ll monoclonal antibody described in Claim 17∼19:
(1) Application in the generation of drugs targeting PD-L1 or PD-1;
(2) Applications in the generation of drugs for the treatment of tumors;
(3) Applications in the generation of drugs for the treatment of immunodegenerative diseases;
(4) Application in the treatment of diseases targeted by PD-L1 or PD-1;
(5) Applications in the treatment of tumors or immunodegenerative diseases.

24. Drug or detection reagent containing any of the De-ADCC/CDC activity anti-PD-L1 monoclonal antibodies described in claim 17∼19.

25. The De-ADCC/CDC activity anti-PD-1 monoclonal antibody is **characterized by** the insertion of a flexible amino acid sequence, blocking the mechanical stress transmit from variable area to constant region after the combining of antigen and antibody. The Fc and/or complement binding sites in heavy chain of the antibody is not sufficiently exposed to Fc receptor and/or complement without this stress transmission. The insertion of the flexible sequence can weaken the interaction between antibody and NK cells, macrophages or neutrophils, which expressing IgG Fc receptors or complement, leading to the reduced or even eliminated ADCC and CDC activity.

26. Monoclonal antibodies, as described in claim 25, are **characterized by** the insertion of a flexible amino acid sequence between the CDR3 and CH2 region of the heavy chain of IgG1 antibody to block the mechanical stress transmit from variable area to constant region after the combining of antigen and antibody. The amino acid sequences of the heavy chain and the light chain of the original Pembrolizumab are shown in SEQ ID NO.5 and 6 respectively.

27. The monoclonal antibodies described in Claim 25 are **characterized by** the full length amino acid sequence of light chain contains as shown in SEQ ID NO.6, and the full length amino acid sequence of heavy chain contains as shown in SEQ ID NO.7.

28. Any genes coding De-ADCC/CDC activity anti-PD-1 monoclonal antibodies as described in claims 25∼27.

29. The biomaterial containing the gene of claim 28, the term "biomaterial" is an expression box, an expression vector, an engineered bacteria or a cell.

30. Any of the following applications of the gene described in Claim 28 or the biomaterial described in claim 29:
(1) Application in the generation of drugs targeting PD-L1 or PD-1;
(2) Application in the treatment of diseases targeted by PD-L1 or PD-1;
(3) Applications in the treatment of tumors or immunodegenerative diseases.

31. Any of the following applications of the De-ADCC/CDC activity anti-PD-1 monoclonal antibody described in Claim 25∼27:
(1) Application in the generation of drugs targeting PD-L1 or PD-1;
(2) Applications in the generation of drugs for the treatment of tumors;
(3) Applications in the generation of drugs for the treatment of immunodegenerative diseases;
(4) Application in the treatment of diseases targeted by PD-L1 or PD-1;
(5) Applications in the treatment of tumors or immunodegenerative diseases.

32. Drug or detection reagent containing any of the De-ADCC/CDC activity anti-PD-1 monoclonal antibodies described in claim 25∼27.

33. The De-ADCC/CDC activity anti-CTLA-4 monoclonal antibody is **characterized by** the insertion of a flexible amino acid sequence in the constant region of Ipilimumab, blocking the mechanical stress transmit from variable area to constant region after the combining of antigen and antibody. The Fc and/or complement binding sites in heavy chain of the antibody is not sufficiently exposed to Fc receptor and/or complement without this stress transmission. The insertion of the flexible sequence can weaken the interaction between antibody and NK cells, macrophages or neutrophils, which expressing IgG Fc receptors or complement, leading to the reduced or even eliminated ADCC and CDC activity.

34. Monoclonal antibodies, as described in claim 33, are **characterized by** the insertion of a flexible amino acid sequence between the CDR3 and CH2 region of the heavy chain of Ipilimumab to block the mechanical stress transmit from variable area to constant region after the combining of antigen and antibody. The amino acid sequences of the heavy chain and the light chain of the original Ipilimumab are shown in SEQ ID NO.8 and 9 respectively.

35. The De-ADCC/CDC activity anti-CTLA-4 monoclonal antibodies described in Claim 34 are **characterized by** the full length amino acid sequence of light chain contains as shown in SEQ ID NO.9, and the full length amino acid sequence of heavy chain contains as shown in SEQ ID NO.10.

36. Any genes coding De-ADCC/CDC activity anti-CTLA-4 monoclonal antibodies as described in claims 33-35.

37. The biomaterial containing the gene of claim 36, the term "biomaterial" is an expression box, an expression vector, an engineered bacteria or a cell.

38. Any of the following applications of the gene described in Claim 36 or the biomaterial described in claim 37:
(1) Application in the generation of drugs targeting CTLA-4;
(2) Application in the treatment of diseases targeted by CTLA-4;
(3) Applications in the treatment of tumors or improvement of the immunity.

39. Any of the following applications of the De-ADCC/CDC activity anti-CTLA-4 monoclonal antibody described in Claim 33∼35:
(1) Application in the generation of drugs targeting CTLA-4;
(2) Applications in the generation of drugs for the treatment of tumors;
(3) Applications in the generation of drugs for the improvement of the immunity;
(4) Application in the treatment of diseases targeted by CTLA-4;
(5) Applications in the treatment of tumors or immunodegenerative diseases.

40. Drug or detection reagent containing any of the De-ADCC/CDC activity anti-CTLA-4 monoclonal antibodies described in claim 33-35.
